(19) Europäisches Patentamt
European Patent Office
Office européen des brevets

(11) Veröffentlichungsnummer: **0 403 891 A1**

# (12) EUROPÄISCHE PATENTANMELDUNG

(21) Anmeldenummer: **90110853.0**

(22) Anmeldetag: **08.06.90**

(51) Int. Cl.[5]: **C07D 207/44, C07D 209/46, A01N 43/36, A01N 43/38**

(30) Priorität: **21.06.89 DE 3920271**

(43) Veröffentlichungstag der Anmeldung: **27.12.90 Patentblatt 90/52**

(84) Benannte Vertragsstaaten: **BE CH DE FR GB IT LI NL**

(71) Anmelder: **BAYER AG**

**D-5090 Leverkusen 1 Bayerwerk(DE)**

(72) Erfinder: **Elbe,Hans-Ludwig, Dr.**
**Dasnöckel 59**
**D-5600 Wuppertal 11(DE)**
Erfinder: **Marhold, Albrecht, Dr.**
**Carl-Duisberg-Strasse 329**
**D-5090 Leverkusen 1(DE)**
Erfinder: **Lürssen, Klaus, Dr.**
**August-Kirspel-Strasse 145**
**D-5060 Bergisch Gladbach 2(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch Gladbach 2(DE)**
Erfinder: **Krauskopf, Birgit, Dr.**
**Kicke 19**
**D-5060 Bergisch Gladbach 1(DE)**

## (54) N-Aryl-Stickstoffheterocyclen.

(57) Die Erfindung betrifft neue N-Aryl-Stickstoffheterocyclen der Formel (I)

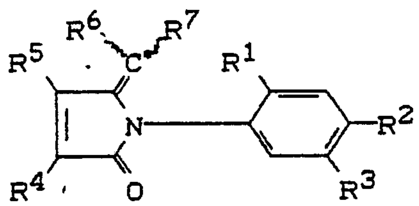

(I)

mehrere Verfahren sowie neue Zwischenprodukte zu deren Herstellung und ihre Verwendung als Herbizide und Pflanzenwuchsregulatoren.

EP 0 403 891 A1

## N-Aryl-Stickstoffheterocyclen

Die vorliegende Erfindung betrifft neue N-Aryl-Stickstoffheterocyclen, mehrere Verfahren sowie neue Zwischenprodukte zu ihrer Herstellung sowie ihre Verwendung als Herbizide und Pflanzenwuchsregulatoren.

Es ist bekannt, daß bestimmte N-Aryl-Stickstoffheterocyclen herbizide Eigenschaften aufweisen (vgl. z.B. US-P 3992189; EP-A 305333; JP-A 63-39859; zit. in Chem. Abstracts 109, 37735p). Die herbizide Wirkung der vorbekannten Verbindungen ist jedoch, ebenso wie ihre Verträglichkeit gegenüber wichtigen Kulturpflanzen, nicht in allen Anwendungsgebieten völlig zufriedenstellend.

Es wurden nun die neuen N-Aryl-Stickstoffheterocyclen der allgemeinen Formel (I)

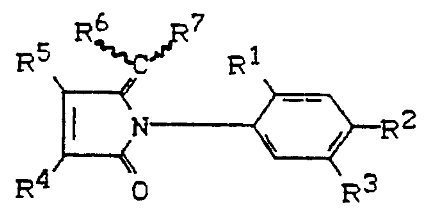

(I)

in welcher

$R^1$ für Wasserstoff oder Halogen steht,

$R^2$ für Cyano, Nitro, Fluor, Chlor, Brom, Jod, Alkyl, Halogenalkyl, Alkoxy, Halogenalkoxy, Alkylthio oder Halogenalkylthio steht.

$R^3$ für Halogen, Hydroxy, Mercapto oder für einen jeweils gegebenenfalls substituierten Rest aus der Reihe Alkoxy, Alkenyloxy, Alkinyloxy, Cycloalkyloxy, Alkylthio, Alkenylthio, Alkinylthio oder Cycloalkylthio steht,

$R^4$ für Wasserstoff, Halogen oder Alkyl steht,

$R^5$ für Wasserstoff, Halogen, Alkyl oder zusammen mit $R^4$ für Alkandiyl steht,

$R^6$ für Wasserstoff oder Alkyl steht und

$R^7$ für Wasserstoff oder Alkyl oder zusammen mit $R^6$ für Alkandiyl steht,

mit der Maßgabe, daß $R^2$ nur dann für Chlor steht, wenn entweder

(a) $R^1$ für Wasserstoff steht oder wenigstens einer der Reste $R^4$, $R^5$, $R^6$, $R^7$ für Alkyl steht und gleichzeitig

$R^3$ für Halogen, Mercapto oder für einen jeweils gegebenenfalls substituierten Rest aus der Reihe Alkylthio, Alkenylthio, Alkinylthio, Cycloalkylthio steht, oder

(b) $R^3$ für Halogen steht oder (in der Bedeutung "gegebenenfalls substituiertes Alkoxy") für Alkoxyalkoxyalkoxy steht, gefunden.

Die Verbindungen der Formel (I) können gegebenenfalls in verschiedenen stereoisomeren Formen auftreten. Die Erfindung betrifft sowohl die einzelnen möglichen Stereoisomeren als auch deren mögliche Gemische.

Weiter wurde gefunden, daß man die neuen N-Aryl-Stickstoffheterocyclen der allgemeinen Formel (I) erhält, wenn man

(a) Ketocarbonsäuren der allgemeinen Formel (II)

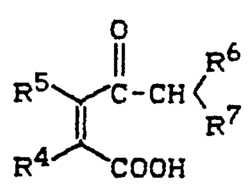

(II)

in welcher

$R^4$, $R^5$, $R^6$ und $R^7$ die oben angegebenen Bedeutungen haben,

mit Arylaminen der allgemeinen Formel (III)

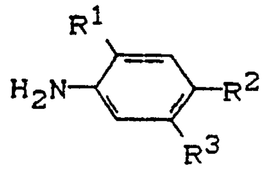

(III)

in welcher
$R^1$, $R^2$ und $R^3$ die oben angegebenen Bedeutungen haben,
gegebenenfalls in Gegenwart eines sauren Katalysators und gegebenenfalls in Gegenwart eines Verdünnungsmittels umsetzt, oder wenn man

(b) N-Aryl-imide der allgemeinen Formel (IV)

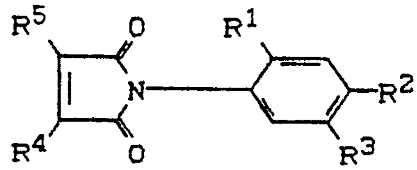

(IV)

in welcher
$R^1$, $R^2$, $R^3$, $R^4$ und $R^5$ die oben angegebenen Bedeutungen haben,
in einer ersten Stufe mit metallorganischen Verbindungen der allgemeinen Formel (V)

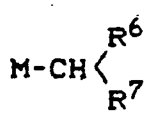

(V)

in welcher
$R^6$ und $R^7$ die oben angegebenen Bedeutungen haben und
M für Lithium oder die Gruppierung MgX steht, worin
X für Chlor, Brom oder Jod steht,
gegebenenfalls in Gegenwart eines Verdünnungsmittels umsetzt und die nach Behandeln mit einer wäßrigen Säure erhaltenen Hydroxyverbindungen der allgemeinen Formel (VI)

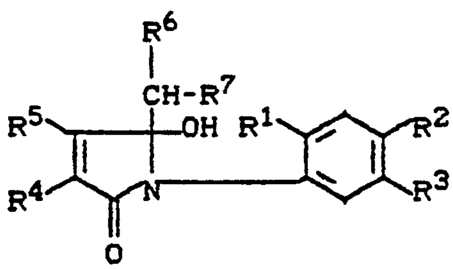

(VI)

in welcher
$R^1$, $R^2$, $R^3$, $R^4$, $R^5$, $R^6$ und $R^7$ die oben angegebenen Bedeutungen haben,
in einer zweiten Stufe nach üblichen Methoden dehydratisiert, oder wenn man

(c) für den Fall, daß in der Formel (I)
$R^3$ für einen gegebenenfalls substituierten Rest aus der Reihe Alkoxy, Alkenyloxy, Alkinyloxy, Cycloalkyloxy, Alkylthio, Alkenylthio, Alkinylthio, Cycloalkylthio steht und
$R^1$, $R^2$, $R^4$, $R^5$, $R^6$ und $R^7$ die oben angegebenen Bedeutungen haben,
Verbindungen der Formel (I),
in welcher
$R^3$ für Hydroxy oder Mercapto steht und
$R^1$, $R^2$, $R^4$, $R^5$, $R^6$ und $R^7$ die oben angegebenen Bedeutungen haben,
mit Alkylierungsmitteln der Formel (VII)

$R^8$-X (VII)

in welcher

$R^8$ für jeweils gegebenenfalls substituiertes Alkyl, Alkenyl, Alkinyl oder Cycloalkyl steht und

X für eine nucleophile Abgangssgruppe steht,

gegebenenfalls in Gegenwart eines Säureakzeptors und gegebenenfalls in Gegenwart eines Verdünnungsmittels umsetzt, oder wenn man

(d) für den Fall, daß in der Formel (I)

$R^3$ für Hydroxy, Mercapto oder für einen jeweils gegebenenfalls substituierten Rest aus der Reihe Alkoxy, Alkenyloxy, Alkinyloxy, Cycloalkyloxy, Alkylthio, Alkenylthio, Alkinylthio, Cycloalkylthio steht und

$R^1$, $R^2$, $R^4$, $R^5$, $R^6$ und $R^7$ die oben angegebenen Bedeutungen haben,

Verbindungen der Formel (I), in welcher

$R^3$ für Halogen steht und

$R^1$, $R^2$, $R^4$, $R^5$, $R^6$ und $R^7$ die oben angegebenen Bedeutungen haben,

mit Verbindungen der Formel (VIII)

H-$R^9$ (VIII)

in welcher

$R^9$ für Hydroxy, Mercapto oder für einen gegebenenfalls substituierten Rest aus der Reihe Alkoxy, Alkenyloxy, Alkinyloxy, Cycloalkyloxy, Alkylthio, Alkenylthio, Alkinylthio, Cycloalkylthio steht

oder mit Metallsalzen von Verbindungen der Formel (VIII)

gegebenenfalls in Gegenwart eines Säureakzeptors und gegebenenfalls in Gegenwart eines Verdünnungsmittels umsetzt.

Die neuen N-Aryl-Stickstoffheterocyclen der allgemeinen Formel (I) zeichnen sich durch starke Herbizidwirkung aus und können auch zur Regulierung des Pflanzenwachstums eingesetzt werden.

Die Erfindung betrifft vorzugsweise Verbindungen der Formel (I), in welcher

$R^1$ für Wasserstoff oder Fluor steht,

$R^2$ für Cyano, Nitro, Fluor, Chlor, Brom, Methyl, Ethyl, Methoxy, Ethoxy, Methylthio, Ethylthio, Halogenalkyl mit 1 oder 2 Kohlenstoffatomen und 1 bis 5 Halogenatomen (insbesondere Fluor- und/oder Chloratomen), Halogenalkoxy mit 1 oder 2 Kohlenstoffatomen und 1 bis 5 Halogenatomen (insbesondere Fluor- und/oder Chloratomen) oder Halogenalkylthio mit 1 oder 2 Kohlenstoffatomen und 1 bis 5 Halogenatomen (insbesondere Fluor- und/oder Chloratomen) steht,

$R^3$ für Fluor, Chlor, Brom, Hydroxy, Mercapto, für jeweils gegebenenfalls substituiertes, geradkettiges oder verzweigtes Alkoxy oder Alkylthio mit jeweils bis zu 8 Kohlenstoffatomen steht, wobei als Substituenten im Alkylteil jeweils vorzugsweise in Frage kommen: Fluor, Chlor, Brom, Cyano, Carboxy, $C_1$-$C_4$-Alkoxy, $C_1$-$C_4$-Alkoxy-$C_1$-$C_4$-alkoxy, $C_1$-$C_4$-Alkylthio, $C_1$-$C_4$-Alkyl-carbonyl, $C_1$-$C_4$-Alkoxy-carbonyl, $C_3$-$C_6$-Cycloalkyloxy-carbonyl, $C_3$-$C_6$-Cycloalkyl-$C_1$-$C_2$-alkoxy-carbonyl, $C_5$-$C_6$-Cycloalkenyloxy-carbonyl, $C_5$-$C_6$-Cycloalkenyl-$C_1$-$C_2$-alkoxy-carbonyl, Tetrahydrofurylmethoxy-carbonyl; weiterhin

$R^3$ für jeweils gegebenenfalls substituiertes und jeweils gegebenenfalls verzweigtes Alkenyloxy, Alkinyloxy, Cycloalkyloxy, Alkenylthio, Alkinylthio oder Cycloalkylthio mit jeweils bis zu 8 Kohlenstoffatomen steht, wobei als Substituenten jeweils in Frage kommen: Fluor, Chlor, Brom, Cyano, Carboxy, $C_1$-$C_4$-Alkoxy-carbonyl;

$R^4$ für Wasserstoff, Chlor oder für geradkettiges oder verzweigtes Alkyl mit 1 bis 4 Kohlenstoffatomen steht,

$R^5$ für Wasserstoff, Chlor, geradkettiges oder verzweigtes Alkyl mit 1 bis 4 Kohlenstoffatomen steht oder $R^5$ zusammen mit $R^4$ für geradkettiges oder verzweigtes Alkandiyl mit 3 bis 6 Kohlenstoffatomen steht,

$R^6$ für Wasserstoff oder geradkettiges oder verzweigtes Alkyl mit 1 bis 4 Kohlenstoffatomen steht und

$R^7$ für Wasserstoff oder geradkettiges oder verzweigtes Alkyl mit 1 bis 4 Kohlenstoffatomen steht oder $R^7$ zusammen mit $R^6$ für geradkettiges oder verzweigtes Alkandiyl mit 2 bis 7 Kohlenstoffatomen steht,

wobei die oben angegebenen Einschränkungen ("Maßgaben") gelten.

Die Erfindung betrifft insbesondere Verbindungen der Formel (I), in welcher

$R^1$ für Wasserstoff oder Fluor steht,

$R^2$ für Cyano, Nitro, Fluor, Chlor, Brom, Methyl, Trifluormethyl, Methoxy, Trifluormethoxy, Difluormethoxy, Chlordifluormethoxy, Methylthio, Trifluormethylthio, Difluormethylthio oder Chlordifluormethylthio steht,

$R^3$ für Fluor, Chlor, Brom, Hydroxy, Mercapto, für jeweils gegebenenfalls substituiertes Methoxy, Ethoxy, Propoxy, Isopropoxy, Butoxy, Isobutoxy, sec-Butoxy, tert-Butoxy, Pentoxy, Isopentoxy, sec-Pentoxy, tert-Pentoxy, Hexyloxy, Isohexyloxy, Methylthio, Ethylthio, Propylthio, Isopropylthio, Butyl thio, Isobutylthio, sec-Butylthio, tert-Butylthio, Pentylthio, Isopentylthio, sec-Pentylthio, tert-Pentylthio, Hexylthio oder Isohexylthio steht, wobei als Substituenten in Frage kommen: Fluor, Chlor, Cyano, Carboxy, Methoxy, Ethoxy, Propoxy, Isopropoxy, Methoxyethoxy, Ethoxyethoxy, Methylthio, Ethylthio, Propylthio, Isopropylthio, Methylcarbonyl, Ethylcarbonyl, Propylcarbonyl, Isopropylcarbonyl, Butylcarbonyl, Isobutylcarbonyl, sec-Butylcarbonyl, tert-Butylcarbonyl, Methoxycarbonyl, Ethoxycarbonyl, Propoxycarbonyl, Isopropoxycarbonyl, Butoxycarbonyl, Isobutoxycarbonyl, sec-Butoxycarbonyl, tert-Butoxycarbonyl, Cyclopropyloxycarbonyl, Cyclopentyloxycarbonyl, Cyclohexyloxycarbonyl, Cyclopentenyloxycarbonyl, Cyclohexenyloxycarbonyl, Cyclopropylmethoxycarbonyl, Cyclopentylmethoxycarbonyl, Cyclohexylmethoxycarbonyl, Cyclopentenylmethoxycarbonyl, Cyclohexenylmethoxycarbonyl, Tetrahydrofurylmethoxycarbonyl; in welcher weiter

$R^3$ für Allyloxy, 1-Methyl-, 2-Methyl- und 3-Methylallyloxy, für Propargyloxy, 1-Methyl-, 3-Methyl-und 1,1-Dimethyl-propargyloxy, für Cyclopropyloxy, Cyclopentyloxy, Cyclohexyloxy, für Allylthio, 1-Methyl-, 2-Methyl- und 3-Methyl-allylthio, für Propargylthio, 1-Methyl-, 3-Methyl- und 1,1-Dimethylpropargylthio, für Cyclopropylthio, Cyclopentylthio, Cyclohexylthio stehen, welche jeweils gegebenenfalls durch Fluor und/oder Chlor substituiert sind;

$R^4$ für Wasserstoff, Methyl, Ethyl, Propyl oder Isopropyl steht,

$R^5$ für Wasserstoff, Methyl, Ethyl, Propyl oder Isopropyl steht oder zusammen mit $R^4$ für Propan-1,3-diyl, Butan-1,3-diyl, Butan-1,4-diyl oder 2,2-Dimethylpropan-1,3-diyl steht,

$R^6$ für Wasserstoff, Methyl, Ethyl, Propyl, Isopropyl, Butyl, Isobutyl, sec-Butyl oder tert-Butyl steht und

$R^7$ für Wasserstoff, Methyl, Ethyl, Propyl oder zusammen mit $R^6$ für Ethan-1,2-diyl, Propan-1,3-diyl, Butan-1,3-diyl, Butan-1,4-diyl, Pentan-1,4-diyl oder Pentan-1,5-diyl steht,

wobei die oben angegebenen Einschränkungen ("Maßgaben") gelten.

Ganz besonders bevorzugt sind Verbindungen der Formel (I), bei welchen

$R^1$ für Wasserstoff oder Fluor steht,

$R^2$ für Chlor, Cyano oder Methyl steht,

$R^4$ für Methyl steht,

$R^5$ für Methyl steht oder zusammen mit $R^4$ für $-(CH_2)_4$-steht,

$R^6$ und $R^7$ unabhängig voneinander für Wasserstoff, Methyl oder Ethyl stehen und

$R^3$ die oben als bevorzugt bzw. insbesondere bevorzugt angegebenen Bedeutungen hat, wobei die oben angegebenen Einschränkungen ("Maßgaben") gelten.

Beispiele für die Verbindungen der Formel (I) sind in der nachstehenden Tabelle 1 aufgeführt (vgl. auch die Herstellungsbeispiele):

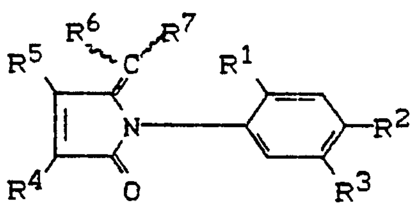

(I)

Tabelle 1: Beispiele für die Verbindungen der Formel (I)

| $R^1$ | $R^2$ | $R^3$ | $R^4$ $R^5$ | $R^6$ | $R^7$ |
|---|---|---|---|---|---|
| H | Cl | $SCH_2CH{=}CH_2$ | $-(CH_2)_4-$ | H | H |
| H | Cl | $SCH_2C{\equiv}CH$ | $-(CH_2)_4-$ | H | H |
| H | Cl | $SCH_2COO$ 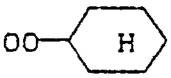 | $-(CH_2)_4-$ | H | H |
| H | Cl | $SCH_2COOC_2H_5$ | $-(CH_2)_4-$ | H | H |
| H | Cl | $SCHC{\equiv}CH$<br>\|<br>$CH_3$ | $-(CH_2)_4-$ | H | H |
| H | Cl | $SCH(CH_3)_2$ | $-(CH_2)_4-$ | H | H |
| H | Cl | $SCH(CH_2F)_2$ | $-(CH_2)_4-$ | H | H |
| H | Cl | $SCHCOOC_4H_9$<br>\|<br>$CH_3$ | $-(CH_2)_4-$ | H | H |
| H | Cl | $SCH_2CN$ | $-(CH_2)_4-$ | H | H |
| H | Cl | $SCH_2CH{=}CHCl$ | $-(CH_2)_4-$ | H | H |
| H | Cl | $SCH_2CH_2OCH_3$ | $-(CH_2)_4-$ | H | H |
| H | Cl | $SCH_2CH_2COOCH_3$ | $-(CH_2)_4-$ | H | H |
| H | Br | $SCH_2CH{=}CH_2$ | $-(CH_2)_4-$ | $CH_3$ | H |
| H | Br | $SCH_2CH{=}CHCH_3$ | $-(CH_2)_4-$ | $CH_3$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| H | Br | $SCH_2CH=CHCl$ | $-(CH_2)_4-$ | | $CH_3$ | H |
| H | Br | $SCH_2C=CH_2$<br>\|<br>$CH_3$ | $-(CH_2)_4-$ | | $CH_3$ | H |
| H | Br | $SCH_2C{\equiv}CH$ | $-(CH_2)_4-$ | | $CH_3$ | H |
| H | Br | $SCH_2C{\equiv}CCH_3$ | $-(CH_2)_4-$ | | $CH_3$ | H |
| H | Br | $SCH_2COO$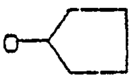 | $-(CH_2)_4-$ | | $CH_3$ | H |
| H | Br | $SCHCOOC_2H_5$<br>\|<br>$CH_3$ | $-(CH_2)_4-$ | | $CH_3$ | H |
| H | Br | $SCH_2COOCH(CH_3)_2$ | $-(CH_2)_4-$ | | $CH_3$ | H |
| H | Br | $SCHCOO$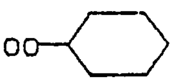<br>\|<br>$CH_3$ | $-(CH_2)_4-$ | | $CH_3$ | H |
| H | Br | $SCH_2COOC_4H_9$ | $-(CH_2)_4-$ | | $CH_3$ | H |
| F | Br | $SCH_2CH=CH_2$ | $-(CH_2)_4-$ | | $CH_3$ | H |
| F | Br | $SCH_2CH=CHCH_3$ | $-(CH_2)_4-$ | | $CH_3$ | H |
| F | Br | $SCH_2CH=CHCl$ | $-(CH_2)_4-$ | | $CH_3$ | H |
| F | Br | $SCH=CH_2$<br>\|<br>$CH_3$ | $-(CH_2)_4-$ | | $CH_3$ | H |
| F | Br | $SCH_2C{\equiv}CH$ | $-(CH_2)_4-$ | | $CH_3$ | H |
| F | Br | $SCH_2C{\equiv}CCH_3$ | $-(CH_2)_4-$ | | $CH_3$ | H |
| F | Br | $SCH_2COOC_4H_9$ | $-(CH_2)_4-$ | | $CH_3$ | H |

<u>Tabelle 1</u> - Fortsetzung

| $R^1$ | $R^2$ | $R^3$ | $R^4$ | $R^5$ | $R^6$ | $R^7$ |
|---|---|---|---|---|---|---|
| F | Br | $SCH_2COO$ 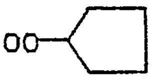 | $-(CH_2)_4-$ | | $CH_3$ | H |
| F | Br | $SCH-COOC_2H_5$<br>\|<br>$CH_3$ | $-(CH_2)_4-$ | | $CH_3$ | H |
| H | Br | $SCH_2CH=CH_2$ | $CH_3$ | $CH_3$ | H | H |
| H | Br | $SCH_2CH=CHCH_3$ | $CH_3$ | $CH_3$ | H | H |
| H | Br | $SCH_2CH=CHCl$ | $CH_3$ | $CH_3$ | H | H |
| H | Br | $SCH=CH_2$<br>\|<br>$CH_3$ | $CH_3$ | $CH_3$ | H | H |
| H | Br | $SCH_2C\equiv CH$ | $CH_3$ | $CH_3$ | H | H |
| H | Br | $SCH_2C\equiv CCH_3$ | $CH_3$ | $CH_3$ | H | H |
| H | Br | $SCH_2COO$ 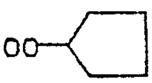 | $CH_3$ | $CH_3$ | H | H |
| H | Br | $SCHCOOC_2H_5$<br>\|<br>$CH_3$ | $CH_3$ | $CH_3$ | H | H |
| H | Br | $SCH_2COOC_4H_9$ | $CH_3$ | $CH_3$ | H | H |
| F | Br | $SCH_2CH=CH_2$ | $CH_3$ | $CH_3$ | H | H |
| F | Br | $SCH_2CH=CHCH_3$ | $CH_3$ | $CH_3$ | H | H |
| F | Br | $SCH_2CH=CHCl$ | $CH_3$ | $CH_3$ | H | H |
| F | Br | $SCH_2COOC_4H_9$ | $CH_3$ | $CH_3$ | H | H |

Tabelle 1 - Fortsetzung

| $R^1$ | $R^2$ | $R^3$ | $R^4$ | $R^5$ | $R^6$ | $R^7$ |
|---|---|---|---|---|---|---|
| F | Br | $SCHCOOC_3H_7$<br>\|<br>$CH_3$ | $CH_3$ | $CH_3$ | H | H |
| F | Br | $SCH_2COO$— 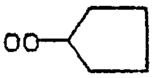 | $CH_3$ | $CH_3$ | H | H |
| F | Br | $SCHCOO$— 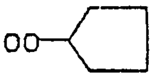<br>\|<br>$CH_3$ | $CH_3$ | $CH_3$ | H | H |
| F | Br | $SCH_2C{\equiv}CH$ | $CH_3$ | $CH_3$ | H | H |
| F | Br | $SCH_2C{\equiv}CCH_3$ | $CH_3$ | $CH_3$ | H | H |
| H | $OCF_3$ | $OCH_2CH{=}CH_2$ | $-(CH_2)_4-$ | | H | H |
| H | $OCF_3$ | $OCH_2C{\equiv}CH$ | $-(CH_2)_4-$ | | H | H |
| H | $OCF_3$ | $OCH_2COOC_2H_5$ | $-(CH_2)_4-$ | | H | H |
| H | $OCF_3$ | $OCH_2CH_2OCH_3$ | $-(CH_2)_4-$ | | H | H |
| H | $SCF_3$ | $OCH_2CH{=}CH_2$ | $-(CH_2)_4-$ | | H | H |
| H | $SCF_3$ | $OCH_2C{\equiv}CH$ | $-(CH_2)_4-$ | | H | H |
| H | $SCF_3$ | $OCH_2COOC_4H_9$ | $-(CH_2)_4-$ | | H | H |
| H | $SCF_3$ | $OCH_2CH_2OC_2H_5$ | $-(CH_2)_4-$ | | H | H |
| F | $OCF_3$ | $OCH_2CH{=}CH_2$ | $-(CH_2)_4-$ | | H | H |
| F | $OCF_3$ | $OCH_2C{\equiv}CCH_3$ | $-(CH_2)_4-$ | | H | H |
| F | $OCF_3$ | $OCH_2COO$— 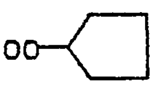 | $-(CH_2)_4-$ | | H | H |

Tabelle 1 - Fortsetzung

| $R^1$ | $R^2$ | $R^3$ | $R^4$ $R^5$ | $R^6$ | $R^7$ |
|---|---|---|---|---|---|
| F | $OCF_3$ | $OCHCOOC_2H_5$<br>$CH_3$ | $-(CH_2)_4-$ | H | H |
| F | $SCF_3$ | $OCH_2CH=CHCl$ | $-(CH_2)_4-$ | H | H |
| F | $SCF_3$ | $OCHC\equiv CH$<br>$CH_3$ | $-(CH_2)_4-$ | H | H |
| F | $SCF_3$ | $OCH_2COOCH(CH_3)_2$ | $-(CH_2)_4-$ | H | H |
| F | $SCF_3$ | $OCH_2CH_2SC_2H_5$ | $-(CH_2)_4-$ | H | H |
| F | $SCF_3$ | OCHCOO 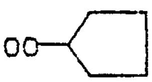<br>$CH_3$ | $-(CH_2)_4-$ | H | H |
| H | $OCF_3$ | $SCH_2CH=CH_2$ | $-(CH_2)_4-$ | H | H |
| H | $OCF_3$ | $SCH_2COOC_2H_5$ | $-(CH_2)_4-$ | H | H |
| H | $OCF_3$ | $SCH_2C\equiv CH$ | $-(CH_2)_4-$ | H | H |
| H | $OCF_3$ | $SCH_2COO$ 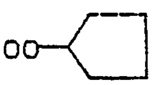 | $-(CH_2)_4-$ | H | H |
| F | $OCF_3$ | $SCH_2CH=CH_2$ | $-(CH_2)_4-$ | H | H |
| F | $OCF_3$ | $SCH_2C\equiv CH$ | $-(CH_2)_4-$ | H | H |
| F | $OCF_3$ | $SCH_2COO$ 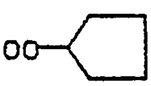 | $-(CH_2)_4-$ | H | H |
| F | $OCF_3$ | $SCHCOOC_2H_5$<br>$CH_3$ | $-(CH_2)_4-$ | H | H |

Tabelle 1 - Fortsetzung

| $R^1$ | $R^2$ | $R^3$ | $R^4$ | $R^5$ | $R^6$ | $R^7$ |
|---|---|---|---|---|---|---|
| H | $SCF_3$ | $SCH_2CH=CH_2$ | $-(CH_2)_4-$ | | H | H |
| H | $SCF_3$ | $SCH_2C\equiv CH$ | $-(CH_2)_4-$ | | H | H |
| H | $SCF_3$ | $SCH_2COOC_2H_5$ | $-(CH_2)_4-$ | | H | H |
| H | $SCF_3$ | $SCH_2CH_2SC_2H_5$ | $-(CH_2)_4-$ | | H | H |
| H | $SCF_3$ | $SCH_2CH_2OC_2H_5$ | $-(CH_2)_4-$ | | H | H |
| F | $SCF_3$ | $SCH_2CH_2SCH_3$ | $-(CH_2)_4-$ | | H | H |
| F | $SCF_3$ | $SCH_2CH=CHCl$ | $-(CH_2)_4-$ | | H | H |
| F | $SCF_3$ | $SCH_2C\equiv CCH_3$ | $-(CH_2)_4-$ | | H | H |
| F | $SCF_3$ | $SCH_2COOC_4H_9$ | $-(CH_2)_4-$ | | H | H |
| F | $SCF_3$ | $SCH_2COO$ 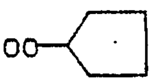 | $-(CH_2)_4-$ | | H | H |
| F | $SCF_3$ | $SCHCOOC_2H_5$<br>$CH_3$ | $-(CH_2)_4-$ | | H | H |
| H | $OCHF_2$ | $OCH_2CH=CH_2$ | $-(CH_2)_4-$ | | H | H |
| H | $OCHF_2$ | $OCH_2C\equiv CH$ | $-(CH_2)_4-$ | | H | H |
| H | $OCHF_2$ | $OCH_2COOC_4H_9$ | $-(CH_2)_4-$ | | H | H |
| H | $OCHF_2$ | $OCH_2COO$ 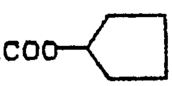 | $-(CH_2)_4-$ | | H | H |
| H | $OCHF_2$ | $OCHCOOC_2H_5$<br>$CH_3$ | $-(CH_2)_4-$ | | H | H |
| F | $OCHF_2$ | $OCH_2CH=CH_2$ | $-(CH_2)_4-$ | | H | H |

<u>Tabelle 1</u> - Fortsetzung

| $R^1$ | $R^2$ | $R^3$ | $R^4$ | $R^5$ | $R^6$ | $R^7$ |
|---|---|---|---|---|---|---|
| F | $OCHF_2$ | $OCH_2C{\equiv}CCH_3$ | $-(CH_2)_4-$ | | H | H |
| F | $OCHF_2$ | $OCH_2COOC_3H_7$ | $-(CH_2)_4-$ | | H | H |
| F | $OCHF_2$ | $OCH_2COO$ 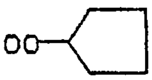 | $-(CH_2)_4-$ | | H | H |
| F | $OCHF_2$ | $OCHCOOCH_3$<br>$CH_3$ | $-(CH_2)_4-$ | | H | H |
| H | $SCHF_2$ | $OCH_2CH{=}CHCH_3$ | $-(CH_2)_4-$ | | H | H |
| H | $SCHF_2$ | $OCH_2C{\equiv}CCH_3$ | $-(CH_2)_4-$ | | H | H |
| H | $SCHF_2$ | $OCH_2COO$ 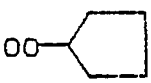 | $-(CH_2)_4-$ | | H | H |
| H | $SCHF_2$ | $OCHCOOC_2H_5$<br>$CH_3$ | $-(CH_2)_4-$ | | H | H |
| F | $SCHF_2$ | $OCH_2CH{=}CHCl$ | $-(CH_2)_4-$ | | H | H |
| F | $SCHF_2$ | $OCH_2C{\equiv}CH$ | $-(CH_2)_4-$ | | H | H |
| F | $SCHF_2$ | $OCH_2COOC_4H_9$ | $-(CH_2)_4-$ | | H | H |
| F | $SCHF_2$ | $OCHCOOC_2H_5$<br>$CH_3$ | $-(CH_2)_4-$ | | H | H |
| H | $OCHF_2$ | $OCH_2CH{=}CH_2$ | $CH_3$ | $CH_3$ | $CH_3$ | H |
| H | $OCHF_2$ | $SCH_2CH{=}CH_2$ | $-(CH_2)_4-$ | | H | H |
| H | $OCHF_2$ | $SCH_2C{\equiv}CH$ | $-(CH_2)_4-$ | | H | H |
| H | $OCHF_2$ | $SCH_2COOC_4H_9$ | $-(CH_2)_4-$ | | H | H |

Tabelle 1 - Fortsetzung

| $R^1$ | $R^2$ | $R^3$ | $R^4$ | $R^5$ | $R^6$ | $R^7$ |
|---|---|---|---|---|---|---|
| H | $OCHF_2$ | $SCH(CH_3)COOCH_3$ | $-(CH_2)_4-$ | | H | H |
| F | $OCHF_2$ | $SCH_2CH=CHCl$ | $-(CH_2)_4-$ | | H | H |
| F | $OCHF_2$ | $SCH_2C\equiv CCH_3$ | $-(CH_2)_4-$ | | H | H |
| F | $OCHF_2$ | $SCH_2COOC_3H_7$ | $-(CH_2)_4-$ | | H | H |
| F | $OCHF_2$ | $SCH_2COO$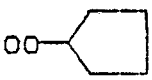 | $-(CH_2)_4-$ | | H | H |
| H | $SCHF_2$ | $SCH_2CH=CHCH_3$ | $-(CH_2)_4-$ | | H | H |
| H | $SCHF_2$ | $SCH_2C\equiv CH$ | $-(CH_2)_4-$ | | H | H |
| H | $SCHF_2$ | $SCH_2COOC_4H_9$ | $-(CH_2)_4-$ | | H | H |
| H | $SCHF_2$ | $SCH(CH_3)COOC_2H_5$ | $-(CH_2)_4-$ | | H | H |
| F | $SCHF_2$ | $SCH_2CH=CH_2$ | $-(CH_2)_4-$ | | H | H |
| F | $SCHF_2$ | $SCH_2C\equiv CH$ | $-(CH_2)_4-$ | | H | H |
| F | $SCHF_2$ | $SCH_2CH_2SC_2H_5$ | $-(CH_2)_4-$ | | H | H |
| F | $SCHF_2$ | $SCH_2CN$ | $-(CH_2)_4-$ | | H | H |
| F | $SCHF_2$ | $SCH_2COOH$ | $-(CH_2)_4-$ | | H | H |
| F | $SCHF_2$ | $SCH(CH_3)COOC_2H_5$ | $-(CH_2)_4-$ | | H | H |
| H | CN | $OCH_2CH=CH_2$ | $-(CH_2)_4-$ | | H | H |
| H | CN | $OCH_2C\equiv CH$ | $-(CH_2)_4-$ | | H | H |

Tabelle 1 - Fortsetzung

| $R^1$ | $R^2$ | $R^3$ | $R^4$ | $R^5$ | $R^6$ | $R^7$ |
|---|---|---|---|---|---|---|
| H | CN | $OCH_2C\equiv CCH_3$ | $-(CH_2)_4-$ | | H | H |
| H | CN | $SCH_2CH=CH_2$ | $-(CH_2)_4-$ | | H | H |
| H | CN | $SCH_2CH=CHCH_3$ | $-(CH_2)_4-$ | | H | H |
| H | CN | $SCH_2CH=CHCl$ | $-(CH_2)_4-$ | | H | H |
| H | CN | $SCH_2COOC_2H_5$ | $-(CH_2)_4-$ | | H | H |
| H | CN | $SCHC\equiv CH$<br>$CH_3$ | $-(CH_2)_4-$ | | H | H |
| F | CN | $OCH_2CH=CHCl$ | $-(CH_2)_4-$ | | H | H |
| F | CN | $OCH_2C\equiv CH$ | $-(CH_2)_4-$ | | H | H |
| F | CN | $OCH_2C\equiv CCH_3$ | $-(CH_2)_4-$ | | H | H |
| F | CN | $OCH_2COOC_4H_9$ | $-(CH_2)_4-$ | | H | H |
| F | CN | $OCHC\equiv CH$<br>$CH_3$ | $-(CH_2)_4-$ | | H | H |
| F | CN | $SCH_2CH=CHCH_3$ | $-(CH_2)_4-$ | | H | H |
| F | CN | $SCH_2C\equiv CH$ | $-(CH_2)_4$ | | H | H |
| F | CN | $SCH_2C=CH_2$<br>Cl | $-(CH_2)_4-$ | | H | H |
| F | CN | $SCH_2C=CH_2$<br>$CH_3$ | $-(CH_2)_4-$ | | H | H |
| F | CN | $SCH_2COO$ 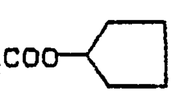 | $-(CH_2)_4-$ | | H | H |

<u>Tabelle 1</u> - Fortsetzung

| $R^1$ | $R^2$ | $R^3$ | $R^4$ $R^5$ | $R^6$ | $R^7$ |
|---|---|---|---|---|---|
| F | CN | $SCH_2COOCH_2$ 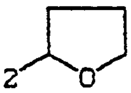 | $-(CH_2)_4-$ | H | H |
| F | CN | $SCH_2CN$ | $-(CH_2)_4-$ | H | H |
| F | CN | $SCH_2CH_2COOC_2H_5$ | $-(CH_2)_4-$ | H | H |
| H | CN | $OCH_2C{\equiv}CH$ | $-CH_2CHCH_2CH_2-$<br>$CH_3$ | H | H |
| F | CN | $OCH_2COOC_2H_5$ | $-CH_2CHCH_2CH_2-$<br>$CH_3$ | H | H |
| H | CN | $OCH_2CH_2OC_2H_5$ | $-CHCH_2CH_2CH_2-$<br>$CH_3$ | H | H |
| F | CN | $SCH_2CH{=}CHCl$ | $-CH_2CH_2CHCH_2-$<br>$CH_3$ | H | H |
| F | CN | $SCHC{\equiv}CH$<br>$CH_3$ | $-CH_2CHCH_2CH_2-$<br>$CH_3$ | H | H |
| H | CN | $SCH(CH_2F)_2$ | $-CH_2CHCH_2CH_2-$<br>$CH_3$ | H | H |
| F | CN | $SCH_2COO$ 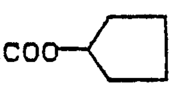 | $-CH_2CHCH_2CH_2-$<br>$CH_3$ | H | H |
| F | CN | $SCH_2CH_2CN$ | $-CH_2CHCH_2CH_2-$<br>$CH_3$ | H | H |

Tabelle 1 - Fortsetzung

| $R^1$ | $R^2$ | $R^3$ | $R^4$ | $R^5$ | $R^6$ | $R^7$ |
|---|---|---|---|---|---|---|
| F | CN | $O(CH_2CH_2O)_2C_2H_5$ | $-CH_2CHCH_2CH_2-$<br>$CH_3$ | | H | H |
| H | CN | $O(CH_2CH_2O)_2CH_3$ | $-CH_2CHCH_2CH_2-$<br>$CH_3$ | | H | H |
| F | CN | $SCH_2CH=CHCl$ | $-CH_2CHCH_2CH_2-$<br>$CH_3$ | | H | H |
| F | CN | $OCHF_2$ | $-CH_2CHCH_2CH_2-$<br>$CH_3$ | | H | H |
| H | Cl | $SCH_2CH=CH_2$ | $-CH_2CHCH_2CH_2-$<br>$CH_3$ | | H | H |
| H | Cl | $SCH_2C\equiv CH$ | $-CH_2CHCH_2CH_2-$<br>$CH_3$ | | H | H |
| H | Cl | $SCH_2COOC_4H_9$ | $-CH_2CHCH_2CH_2-$<br>$CH_3$ | | H | H |
| F | CN | $OCH_2CH=CHCH_3$ | $-(CH_2)_4-$ | | $CH_3$ | H |
| F | CN | $OCH_2C\equiv CH$ | $-(CH_2)_4-$ | | $CH_3$ | H |
| F | CN | $OCH_2CH_2OC_2H_5$ | $-(CH_2)_4-$ | | $CH_3$ | H |
| F | CN | $OCH_2COOC_3H_7$ | $-(CH_2)_4-$ | | $CH_3$ | H |
| F | CN | $OCH_2COO$ 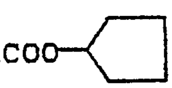 | $-(CH_2)_4-$ | | $CH_3$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| F | CN | $OCHCOOC_2H_5$<br>$CH_3$ | $-(CH_2)_4-$ | | $CH_3$ | H |
| F | CN | $SCH_2CH{\equiv}CH$ | $-(CH_2)_4-$ | | $CH_3$ | H |
| F | CN | $SCHC{\equiv}CH$<br>$CH_3$ | $-(CH_2)_4-$ | | $CH_3$ | H |
| F | CN | $SCH_2CH{=}CHCl$ | $-(CH_2)_4-$ | | $CH_3$ | H |
| F | CN | $SCH_2COOCH(CH_3)_2$ | $-(CH_2)_4-$ | | $CH_3$ | H |
| F | CN | $SCH_2COO$– 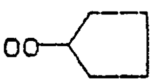 | $-(CH_2)_4-$ | | $CH_3$ | H |
| F | CN | $SCHCN$<br>$CH_3$ | $-(CH_2)_4-$ | | $CH_3$ | H |
| F | CN | $SCH_2CH_2SCH_3$ | $-(CH_2)_4-$ | | $CH_3$ | H |
| F | CN | $SCHCOOCH_3$<br>$CH_3$ | $-(CH_2)_4-$ | | $CH_3$ | H |
| H | CN | $OCH_2C{\equiv}CH$ | $-(CH_2)_4-$ | | $CH_3$ | H |
| H | CN | $OCH_2CH{=}CHCl$ | $-(CH_2)_4-$ | | $CH_3$ | H |
| H | CN | $OCH_2COOC_4H_9$ | $-(CH_2)_4-$ | | $CH_3$ | H |
| H | CN | $O(CH_2CH_2O)_2C_2H_5$ | $-(CH_2)_4-$ | | $CH_3$ | H |
| H | CN | $OCHCOOC_2H_5$<br>$CH_3$ | $-(CH_2)_4-$ | | $CH_3$ | H |
| H | CN | $SCH_2C{\equiv}CCH_3$ | $-(CH_2)_4-$ | | $CH_3$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| H | CN | $SCH_2CH{=}CHCH_3$ | $-(CH_2)_4-$ | | $CH_3$ | H |
| H | CN | $SCH_2COO$ 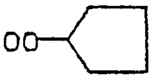 | $-(CH_2)_4-$ | | $CH_3$ | H |
| H | CN | $SCH(CH_2F)_2$ | $-(CH_2)_4-$ | | $CH_3$ | H |
| H | CN | $SCH_2CH_2CN$ | $-(CH_2)_4-$ | | $CH_3$ | H |
| H | Cl | $SCH_2CH{=}CH_2$ | $-(CH_2)_4-$ | | $CH_3$ | H |
| H | Cl | $SCH_2C{\equiv}CCH_3$ | $-(CH_2)_4-$ | | $CH_3$ | H |
| H | Cl | $SCH(CH_3)C{\equiv}CH$ | $-(CH_2)_4-$ | | $CH_3$ | H |
| H | Cl | $SCH_2COO$ 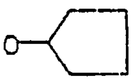 | $-(CH_2)_4-$ | | $CH_3$ | H |
| H | Cl | $SCH(CH_2F)_2$ | $-(CH_2)_4-$ | | $CH_3$ | H |
| H | Cl | $SCH_2COOC_4H_9$ | $-(CH_2)_4-$ | | $CH_3$ | H |
| H | Cl | $SCH_2CN$ | $-(CH_2)_4-$ | | $CH_3$ | H |
| H | Cl | $SCH_2CH_2SCH_3$ | $-(CH_2)_4-$ | | $CH_3$ | H |
| H | Cl | $SCH(CH_3)COOC_2H_5$ | $-(CH_2)_4-$ | | $CH_3$ | H |
| F | Cl | $SCH_2CH{=}CH_2$ | $-(CH_2)_4-$ | | $CH_3$ | H |
| F | Cl | $SCH_2C{\equiv}CH$ | $-(CH_2)_4-$ | | $CH_3$ | H |
| F | Cl | $SCH(CH_3)C{\equiv}CH$ | $-(CH_2)_4-$ | | $CH_3$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| F | Cl | $SCH_2COOC_4H_9$ | $-(CH_2)_4-$ | | $CH_3$ | H |
| F | Cl | $SCH(CH_3)COOC_2H_5$ | $-(CH_2)_4-$ | | $CH_3$ | H |
| F | CN | $SCH_2CH=CHCH_3$ | $-(CH_2)_4-$ | | $CH_3$ | H |
| F | CN | $SCH_2C\equiv CCH_3$ | $-(CH_2)_4-$ | | $CH_3$ | H |
| F | CN | $SCH_2COOC_3H_7$ | $-(CH_2)_4-$ | | $CH_3$ | H |
| F | CN | $SCH(CH_2F)_2$ | $-(CH_2)_4-$ | | $CH_3$ | H |
| F | CN | $SCH(CH_3)COOCH_3$ | $-(CH_2)_4-$ | | $CH_3$ | H |
| F | CN | $OCH_2CH=CH_2$ | $-(CH_2)_4-$ | | $CH_3$ | H |
| F | CN | $OCH_2C\equiv CH$ | $-(CH_2)_4-$ | | $CH_3$ | H |
| F | CN | $OCHF_2$ | $-(CH_2)_4-$ | | $CH_3$ | H |
| F | CN | $OCF_2CHF_2$ | $-(CH_2)_4-$ | | $CH_3$ | H |
| F | CN | $OCHFCF_3$ | $-(CH_2)_4-$ | | $CH_3$ | H |
| F | CN | $OCF_2CHFCl$ | $-(CH_2)_4-$ | | $CH_3$ | H |
| F | CN | $OCH_2CH_2OC_2H_5$ | $-(CH_2)_4-$ | | $CH_3$ | H |
| F | CN | $OCH_2COOC_4H_9$ | $-(CH_2)_4-$ | | $CH_3$ | H |
| F | CN | $OCH_2COO$ 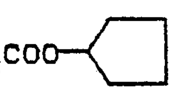 | $-(CH_2)_4-$ | | $CH_3$ | H |
| F | CN | $OCH(CH_3)COOC_2H_5$ | $-(CH_2)_4-$ | | $CH_3$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| H | CN | $OCH_2CH=CHCl$ | $-(CH_2)_4-$ | | $CH_3$ | H |
| H | CN | $OCH_2C\equiv CH$ | $-(CH_2)_4-$ | | $CH_3$ | H |
| H | CN | $OCH_2COOC_3H_7$ | $-(CH_2)_4-$ | | $CH_3$ | H |
| H | CN | $O(CH_2CH_2O)_2CH_3$ | $-(CH_2)_4-$ | | $CH_3$ | H |
| H | CN | $SCH_2CN$ | $-(CH_2)_4-$ | | $CH_3$ | H |
| H | CN | $SCH_2C\equiv CH$ | $-(CH_2)_4-$ | | $CH_3$ | H |
| H | CN | $SCH_2COO$ 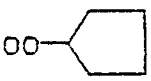 | $-(CH_2)_4-$ | | $CH_3$ | H |
| F | Cl | $SCH_2CH=CH_2$ | $-(CH_2)_4-$ | | $CH_3$ | H |
| F | Cl | $SCH_2CH=CHCl$ | $-(CH_2)_4-$ | | $CH_3$ | H |
| F | Cl | $SCH_2CH=CHCH_3$ | $-(CH_2)_4-$ | | $CH_3$ | H |
| F | Cl | $SCH_2C\equiv CH$ | $-(CH_2)_4-$ | | $CH_3$ | H |
| F | Cl | $SCH_2C\equiv CCH_3$ | $-(CH_2)_4-$ | | $CH_3$ | H |
| F | Cl | $SCH_2CH_2SCH_3$ | $-(CH_2)_4-$ | | $CH_3$ | H |
| F | Cl | $SCH_2COOC_3H_7$ | $-(CH_2)_4-$ | | $CH_3$ | H |
| F | Cl | $SCH_2COO$ 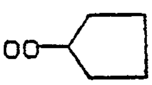 | $-(CH_2)_4-$ | | $CH_3$ | H |
| F | Cl | $SCH_2COOCH_2$ 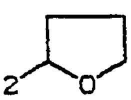 | $-(CH_2)_4-$ | | $CH_3$ | H |
| F | Cl | $SCH_2CH_2COOCH_3$ | $-(CH_2)_4-$ | | $CH_3$ | H |
| F | Cl | $SCH(CH_2F)_2$ | $-(CH_2)_4-$ | | $CH_3$ | H |

Tabelle 1 - Fortsetzung

| $R^1$ | $R^2$ | $R^3$ | $R^4$ $R^5$ | $R^6$ | $R^7$ |
|---|---|---|---|---|---|
| F | CN | $OCH_2CH{=}CH_2$ | $-(CH_2)_4-$ | $C_2H_5$ | H |
| F | CN | $OCH_2C{\equiv}CH$ | $-(CH_2)_4-$ | $C_2H_5$ | H |
| F | CN | $OCH_2COOC_3H_7$ | $-(CH_2)_4-$ | $C_2H_5$ | H |
| F | CN | $OCH_2CH_2OC_2H_5$ | $-(CH_2)_4-$ | $C_2H_5$ | H |
| F | CN | $O(CH_2CH_2O)_2C_2H_5$ | $-(CH_2)_4-$ | $C_2H_5$ | H |
| F | CN | $OCHF_2$ | $-(CH_2)_4-$ | $C_2H_5$ | H |
| F | CN | $SCH_2CH{=}CH_2$ | $-(CH_2)_4-$ | $C_2H_5$ | H |
| F | CN | $SCH_2C{\equiv}CH$ | $-(CH_2)_4-$ | $C_2H_5$ | H |
| F | CN | $SCH_2C{\equiv}CCH_3$ | $-(CH_2)_4-$ | $C_2H_5$ | H |
| F | CN | $SCHC{\equiv}CH$<br>\|<br>$CH_3$ | $-(CH_2)_4-$ | $C_2H_5$ | H |
| F | CN | $SCH_2CH{=}CHCl$ | $-(CH_2)_4-$ | $C_2H_5$ | H |
| F | CN | $SCH_2COOC_4H_9$ | $-(CH_2)_4-$ | $C_2H_5$ | H |
| F | CN | $SCH_2COO$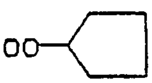 | $-(CH_2)_4-$ | $C_2H_5$ | H |
| F | CN | $SCHCOOCH_3$<br>\|<br>$CH_3$ | $-(CH_2)_4-$ | $C_2H_5$ | H |
| F | CN | $SCH(CH_2F)_2$ | $-(CH_2)_4-$ | $C_2H_5$ | H |
| F | CN | $SCH_2CH_2CN$ | $-(CH_2)_4-$ | $C_2H_5$ | H |
| F | CN | $SCH_2COOCH_2$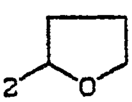 | $-(CH_2)_4-$ | $C_2H_5$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| H | CN | $OCH_2COOC_4H_9$ | $-(CH_2)_4-$ | | $C_2H_5$ | H |
| H | CN | $OCH_2C{\equiv}CH$ | $-(CH_2)_4-$ | | $C_2H_5$ | H |
| H | CN | $SCH_2COO-$ 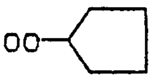 | $-(CH_2)_4-$ | | $C_2H_5$ | H |
| H | CN | $SCHCOOC_2H_5$ with $CH_3$ on the first C | $-(CH_2)_4-$ | | $C_2H_5$ | H |
| H | CN | $OCHF_2$ | $-(CH_2)_4-$ | | $C_2H_5$ | H |
| H | CN | $O(CH_2CH_2O)_2C_2H_5$ | $-(CH_2)_4-$ | | $C_2H_5$ | H |
| H | CN | $SCH_2CH_2COOCH_3$ | $-(CH_2)_4-$ | | $C_2H_5$ | H |
| H | CN | $SCH_2C{\equiv}CCH_3$ | $-(CH_2)_4-$ | | $C_2H_5$ | H |
| H | Cl | $SCH_2CH{=}CH_2$ | $-(CH_2)_4-$ | | $C_2H_5$ | H |
| H | Cl | $SCH_2CH{=}CHCl$ | $-(CH_2)_4-$ | | $C_2H_5$ | H |
| H | Cl | $SCH_2CH{=}CHCH_3$ | $-(CH_2)_4-$ | | $C_2H_5$ | H |
| H | Cl | $SCH_2C(Cl){=}CH_2$ | $-(CH_2)_4-$ | | $C_2H_5$ | H |
| H | Cl | $SCH(CH_3)CH{=}CH_2$ | $-(CH_2)_4-$ | | $C_2H_5$ | H |
| H | Cl | $SCH_2COOC_2H_5$ | $-(CH_2)_4-$ | | $C_2H_5$ | H |
| H | Cl | $SCH(CH_2F)_2$ | $-(CH_2)_4-$ | | $C_2H_5$ | H |
| H | Cl | $SCH_2COO-$ 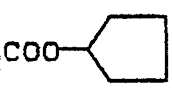 | $-(CH_2)_4-$ | | $C_2H_5$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| H | Cl | $SCHCOOC_2H_5$<br>$CH_3$ | $-(CH_2)_4-$ | | $C_2H_5$ | H |
| H | Cl | $SCH_2CH_2CN$ | $-(CH_2)_4-$ | | $C_2H_5$ | H |
| H | Cl | $SCH_2CH_2COOCH_3$ | $-(CH_2)_4-$ | | $C_2H_5$ | H |
| F | Cl | $SCH_2CH=CH_2$ | $-(CH_2)_4-$ | | $C_2H_5$ | H |
| F | Cl | $SCH_2C\equiv CH$ | $-(CH_2)_4-$ | | $C_2H_5$ | H |
| F | Cl | $SCH_2COOC_4H_9$ | $-(CH_2)_4-$ | | $C_2H_5$ | H |
| F | Cl | $SCH_2CH_2COOCH_3$ | $-(CH_2)_4-$ | | $C_2H_5$ | H |
| F | Cl | $SCH_2COO$ 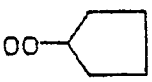 | $-(CH_2)_4-$ | | $C_2H_5$ | H |
| F | Cl | $SCHCOOCH_3$<br>$CH_3$ | $-(CH_2)_4-$ | | $C_2H_5$ | H |
| F | CN | $OCH_2CH=CH_2$ | $-(CH_2)_4-$ | | $C_3H_7$ | H |
| F | CN | $OCH_2C\equiv CH$ | $-(CH_2)_4-$ | | $C_3H_7$ | H |
| F | CN | $OCH_2COOC_2H_5$ | $-(CH_2)_4-$ | | $C_3H_7$ | H |
| F | CN | $OCH_2COO$ 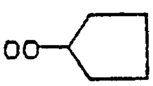 | $-(CH_2)_4-$ | | $C_3H_7$ | H |
| F | CN | $SCHCH=CH_2$<br>$CH_3$ | $-(CH_2)_4-$ | | $C_3H_7$ | H |
| F | CN | $SCH_2CH=CHCl$ | $-(CH_2)_4-$ | | $C_3H_7$ | H |
| F | CN | $SCH_2COOC_4H_9$ | $-(CH_2)_4-$ | | $C_3H_7$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| F | CN | $SCH(CH_3)COOC_2H_5$ | $-(CH_2)_4-$ | | $C_3H_7$ | H |
| F | CN | $SCH_2CH_2COOCH_3$ | $-(CH_2)_4-$ | | $C_3H_7$ | H |
| F | CN | $SCH(CH_2F)_2$ | $-(CH_2)_4-$ | | $C_3H_7$ | H |
| H | CN | $OCH_2CH{=}CHCH_3$ | $-(CH_2)_4-$ | | $C_3H_7$ | H |
| H | CN | $OCH_2CH{=}CHCl$ | $-(CH_2)_4-$ | | $C_3H_7$ | H |
| H | CN | $OCH_2C{\equiv}CCH_3$ | $-(CH_2)_4-$ | | $C_3H_7$ | H |
| H | CN | $OCH_2COOC_4H_9$ | $-(CH_2)_4-$ | | $C_3H_7$ | H |
| H | CN | $OCH_2COOCH_2$- 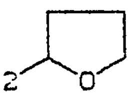 | $-(CH_2)_4-$ | | $C_3H_7$ | H |
| H | CN | $SCH_2C{\equiv}CH$ | $-(CH_2)_4-$ | | $C_3H_7$ | H |
| H | CN | $SCH(CH_3)CH{=}CH_2$ | $-(CH_2)_4-$ | | $C_3H_7$ | H |
| H | CN | $SCH_2COOC_3H_7$ | $-(CH_2)_4-$ | | $C_3H_7$ | H |
| H | CN | $SCH_2CH_2CN$ | $-(CH_2)_4-$ | | $C_3H_7$ | H |
| H | CN | $SCH_2CH_2COOC_2H_5$ | $-(CH_2)_4-$ | | $C_3H_7$ | H |
| H | CN | $SCH(CH_3)COOC_2H_5$ | $-(CH_2)_4-$ | | $C_3H_7$ | H |
| H | Cl | $SCH_2CH{=}CH_2$ | $-(CH_2)_4-$ | | $C_3H_7$ | H |
| H | Cl | $SCH_2CH{=}CHCl$ | $-(CH_2)_4-$ | | $C_3H_7$ | H |

Tabelle 1 - Fortsetzung

| $R^1$ | $R^2$ | $R^3$ | $R^4$ $R^5$ | $R^6$ | $R^7$ |
|---|---|---|---|---|---|
| H | Cl | $SCH_2C=CH_2$<br>Cl | $-(CH_2)_4-$ | $C_3H_7$ | H |
| H | Cl | $SCH_2C\equiv CH$ | $-(CH_2)_4-$ | $C_3H_7$ | H |
| H | Cl | $SCH_2C\equiv CCH_3$ | $-(CH_2)_4-$ | $C_3H_7$ | H |
| H | Cl | $SCHC\equiv CH$<br>$CH_3$ | $-(CH_2)_4-$ | $C_3H_7$ | H |
| H | Cl | $SCH_2COOC_4H_9$ | $-(CH_2)_4-$ | $C_3H_7$ | H |
| H | Cl | $SCH_2COO$ 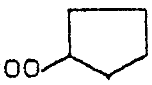 | $-(CH_2)_4-$ | $C_3H_7$ | H |
| H | Cl | $SCHCOOCH_3$<br>$CH_3$ | $-(CH_2)_4-$ | $C_3H_7$ | H |
| F | Cl | $SCH_2CH=CH_2$ | $-(CH_2)_4-$ | $C_3H_7$ | H |
| F | Cl | $SCH_2CH=CHCH_3$ | $-(CH_2)_4-$ | $C_3H_7$ | H |
| F | Cl | $SCH_2C\equiv CH$ | $-(CH_2)_4-$ | $C_3H_7$ | H |
| F | Cl | $SCHC\equiv CH$<br>$CH_3$ | $-(CH_2)_4-$ | $C_3H_7$ | H |
| F | Cl | $SCH_2COOC_3H_7$ | $-(CH_2)_4-$ | $C_3H_7$ | H |
| F | Cl | $SCH_2COOCH_2$ 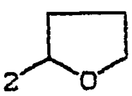 | $-(CH_2)_4-$ | $C_3H_7$ | H |
| F | Cl | $SCH_2CH_2CN$ | $-(CH_2)_4-$ | $C_3H_7$ | H |
| F | Cl | $SCH_2CH_2COOC_2H_5$ | $-(CH_2)_4-$ | $C_3H_7$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| F | Cl | $SCHCOOCH_3$<br>\|<br>$CH_3$ | $-(CH_2)_4-$ | | $C_3H_7$ | H |
| F | CN | $OCH_2CH=CH_2$ | $-(CH_2)_4-$ | | $CH_3$ | $CH_3$ |
| F | CN | $OCH_2C\equiv CH$ | $-(CH_2)_4-$ | | $CH_3$ | $CH_3$ |
| F | CN | $OCH_2COOC_2H_5$ | $-(CH_2)_4-$ | | $CH_3$ | $CH_3$ |
| F | CN | $OCH(CH_2F)_2$ | $-(CH_2)_4-$ | | $CH_3$ | $CH_3$ |
| F | CN | $SCH_2CH=CH_2$ | $-(CH_2)_4-$ | | $CH_3$ | $CH_3$ |
| F | CN | $SCHC\equiv CH$<br>\|<br>$CH_3$ | $-(CH_2)_4-$ | | $CH_3$ | $CH_3$ |
| F | CN | $SCH_2C\equiv CCH_3$ | $-(CH_2)_4-$ | | $CH_3$ | $CH_3$ |
| F | CN | $SCH_2COOC_4H_9$ | $-(CH_2)_4-$ | | $CH_3$ | $CH_3$ |
| F | CN | $SCH_2CH_2CN$ | $-(CH_2)_4-$ | | $CH_3$ | $CH_3$ |
| F | CN | $SCH_2CH_2COOCH_3$ | $-(CH_2)_4-$ | | $CH_3$ | $CH_3$ |
| F | CN | $SCHCOOC_2H_5$<br>\|<br>$CH_3$ | $-(CH_2)_4-$ | | $CH_3$ | $CH_3$ |
| H | CN | $OCH_2CH=CHCH_3$ | $-(CH_2)_4-$ | | $CH_3$ | $CH_3$ |
| H | CN | $OCH_2CH=CH_2$ | $-(CH_2)_4-$ | | $CH_3$ | $CH_3$ |
| H | CN | $OCH_2C\equiv CH$ | $-(CH_2)_4-$ | | $CH_3$ | $CH_3$ |
| H | CN | $OCHF_2$ | $-(CH_2)_4-$ | | $CH_3$ | $CH_3$ |
| H | CN | $OCH(CH_2F)_2$ | $-(CH_2)_4-$ | | $CH_3$ | $CH_3$ |
| H | CN | $OCH_2COOC_4H_9$ | $-(CH_2)_4$ | | $CH_3$ | $CH_3$ |

Tabelle 1 - Fortsetzung

| $R^1$ | $R^2$ | $R^3$ | $R^4$ | $R^5$ | $R^6$ | $R^7$ |
|---|---|---|---|---|---|---|
| H | CN | $SCH_2CH{=}CH_2$ | $-(CH_2)_4-$ | | $CH_3$ | $CH_3$ |
| H | CN | $SCH_2C{\equiv}CH$ | $-(CH_2)_4-$ | | $CH_3$ | $CH_3$ |
| H | CN | $SCH_2COOC_2H_5$ | $-(CH_2)_4-$ | | $CH_3$ | $CH_3$ |
| H | CN | $SCHCOOCH_3$<br>$CH_3$ | $-(CH_2)_4-$ | | $CH_3$ | $CH_3$ |
| H | Cl | $SCH_2CH{=}CH_2$ | $-(CH_2)_4-$ | | $CH_3$ | $CH_3$ |
| H | Cl | $SCH_2C{\equiv}CH$ | $-(CH_2)_4-$ | | $CH_3$ | $CH_3$ |
| H | Cl | $SC(CH_3)_2C{\equiv}CH$ | $-(CH_2)_4-$ | | $CH_3$ | $CH_3$ |
| H | Cl | $SCH_2COOC_2H_5$ | $-(CH_2)_4-$ | | $CH_3$ | $CH_3$ |
| H | Cl | $SCH_2CH_2CN$ | $-(CH_2)_4-$ | | $CH_3$ | $CH_3$ |
| H | Cl | $SCH_2CH_2COOCH_3$ | $-(CH_2)_4-$ | | $CH_3$ | $CH_3$ |
| H | Cl | $SCHCOOC_2H_5$<br>$CH_3$ | $-(CH_2)_4-$ | | $CH_3$ | $CH_3$ |
| F | Cl | $SCH_2CH{=}CHCH_3$ | $-(CH_2)_4-$ | | $CH_3$ | $CH_3$ |
| F | Cl | $SCH_2C{\equiv}CH$ | $-(CH_2)_4-$ | | $CH_3$ | $CH_3$ |
| F | Cl | $SCH_2CH{=}CHCl$ | $-(CH_2)_4-$ | | $CH_3$ | $CH_3$ |
| F | Cl | $SCH_2C{=}CH_2$<br>Cl | $-(CH_2)_4-$ | | $CH_3$ | $CH_3$ |
| F | Cl | $SCHC{\equiv}CH$<br>$CH_3$ | $-(CH_2)_4-$ | | $CH_3$ | $CH_3$ |
| F | Cl | $SCH_2COOC_2H_5$ | $-(CH_2)_4-$ | | $CH_3$ | $CH_3$ |

Tabelle 1 - Fortsetzung

| $R^1$ | $R^2$ | $R^3$ | $R^4$ | $R^5$ | $R^6$ | $R^7$ |
|---|---|---|---|---|---|---|
| F | Cl | $SCH_2COO$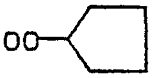 | $-(CH_2)_4-$ | | $CH_3$ | $CH_3$ |
| F | Cl | $SCHCOOCH_3$<br>\|<br>$CH_3$ | $-(CH_2)_4-$ | | $CH_3$ | $CH_3$ |
| F | Cl | $SCH(CH_2F)_2$ | $-(CH_2)_4-$ | | $CH_3$ | $CH_3$ |
| F | Cl | $SCH_2CH_2CN$ | $-(CH_2)_4-$ | | $CH_3$ | $CH_3$ |
| F | Cl | $SCH_2CH_2COOCH_3$ | $-(CH_2)_4-$ | | $CH_3$ | $CH_3$ |
| F | CN | $OCH_2CH=CH_2$ | $CH_3$ | $CH_3$ | H | H |
| F | CN | $OCH_2C\equiv CH$ | $CH_3$ | $CH_3$ | H | H |
| F | CN | $OCHF_2$ | $CH_3$ | $CH_3$ | H | H |
| F | CN | $OCH(CH_2F)_2$ | $CH_3$ | $CH_3$ | H | H |
| F | CN | $OCF_2CHFCl$ | $CH_3$ | $CH_3$ | H | H |
| F | CN | $OCH_2COOC_4H_9$ | $CH_3$ | $CH_3$ | H | H |
| F | CN | $SCH_2CH=CH_2$ | $CH_3$ | $CH_3$ | H | H |
| F | CN | $SCH_2C\equiv CH$ | $CH_3$ | $CH_3$ | H | H |
| F | CN | $SCHC\equiv CH$<br>\|<br>$CH_3$ | $CH_3$ | $CH_3$ | H | H |
| F | CN | $SCH_2CN$ | $CH_3$ | $CH_3$ | H | H |
| F | CN | $SCH_2CH_2CN$ | $CH_3$ | $CH_3$ | H | H |
| F | CN | $SCH_2COOC_3H_7$ | $CH_3$ | $CH_3$ | H | H |

Tabelle 1 - Fortsetzung

| $R^1$ | $R^2$ | $R^3$ | $R^4$ | $R^5$ | $R^6$ | $R^7$ |
|---|---|---|---|---|---|---|
| F | CN | $SCH_2COO$– 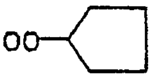 | $CH_3$ | $CH_3$ | H | H |
| H | CN | $OCH_2CH{=}CHCH_3$ | $CH_3$ | $CH_3$ | H | H |
| H | CN | $OCH_2C{\equiv}CCH_3$ | $CH_3$ | $CH_3$ | H | H |
| H | CN | $OCH_2COOC_4H_9$ | $CH_3$ | $CH_3$ | H | H |
| H | CN | $SCH_2C{\equiv}CH$ | $CH_3$ | $CH_3$ | H | H |
| H | CN | $SCH(CH_3)C{\equiv}CH$ | $CH_3$ | $CH_3$ | H | H |
| H | CN | $SCH_2COOC_3H_7$ | $CH_3$ | $CH_3$ | H | H |
| H | CN | $SCH_2CH_2COOCH_3$ | $CH_3$ | $CH_3$ | H | H |
| H | CN | $SCH(CH_3)COOCH_3$ | $CH_3$ | $CH_3$ | H | H |
| H | Cl | $SCH_2CH{=}CH_2$ | $CH_3$ | $CH_3$ | H | H |
| H | Cl | $SCH(CH_3)CH{=}CH_2$ | $CH_3$ | $CH_3$ | H | H |
| H | Cl | $SCH_2CH{=}CHCl$ | $CH_3$ | $CH_3$ | H | H |
| H | Cl | $SCH_2C{\equiv}CH$ | $CH_3$ | $CH_3$ | H | H |
| H | Cl | $SC(CH_3)_2C{\equiv}CH$ | $CH_3$ | $CH_3$ | H | H |
| H | Cl | $SCH_2COOC_3H_7$ | $CH_3$ | $CH_3$ | H | H |
| H | Cl | $SCH_2COO$– 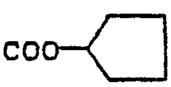 | $CH_3$ | $CH_3$ | H | H |

Tabelle 1 - Fortsetzung

| $R^1$ | $R^2$ | $R^3$ | $R^4$ | $R^5$ | $R^6$ | $R^7$ |
|---|---|---|---|---|---|---|
| H | Cl | $SCH_2CH_2CN$ | $CH_3$ | $CH_3$ | H | H |
| H | Cl | $SCH_2CH_2COOCH_3$ | $CH_3$ | $CH_3$ | H | H |
| H | Cl | $SCHCOOCH_3$<br>$CH_3$ | $CH_3$ | $CH_3$ | H | H |
| F | Cl | $SCH_2CH{=}CH_2$ | $CH_3$ | $CH_3$ | H | H |
| F | Cl | $SCH_2C{=}CH_2$<br>Cl | $CH_3$ | $CH_3$ | H | H |
| F | Cl | $SCH_2C{\equiv}CH$ | $CH_3$ | $CH_3$ | H | H |
| F | Cl | $SCHC{\equiv}CH$<br>$CH_3$ | $CH_3$ | $CH_3$ | H | H |
| F | Cl | $SCH_2CH_2CN$ | $CH_3$ | $CH_3$ | H | H |
| F | Cl | $SCH_2CH_2COOCH_3$ | $CH_3$ | $CH_3$ | H | H |
| F | Cl | $SCH_2COOC_2H_5$ | $CH_3$ | $CH_3$ | H | H |
| F | Cl | $SCH(CH_2F)_2$ | $CH_3$ | $CH_3$ | H | H |
| F | Cl | $SCHCOOCH_3$<br>$CH_3$ | $CH_3$ | $CH_3$ | H | H |
| F | CN | $OCH_2CH{=}CH_2$ | $CH_3$ | $CH_3$ | $CH_3$ | H |
| F | CN | $OCH_2C{\equiv}CH$ | $CH_3$ | $CH_3$ | $CH_3$ | H |
| F | CN | $OCH_2COOC_2H_5$ | $CH_3$ | $CH_3$ | $CH_3$ | H |
| F | CN | $O(CH_2CH_2O)_2C_2H_5$ | $CH_3$ | $CH_3$ | $CH_3$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| F | CN | $OCHCOOCH_3$<br>\|<br>$CH_3$ | $CH_3$ | $CH_3$ | $CH_3$ | H |
| F | CN | $SCH_2CH{=}CHCl$ | $CH_3$ | $CH_3$ | $CH_3$ | H |
| F | CN | $SCH_2C{\equiv}CCH_3$ | $CH_3$ | $CH_3$ | $CH_3$ | H |
| F | CN | $SCH_2CH_2CN$ | $CH_3$ | $CH_3$ | $CH_3$ | H |
| F | CN | $SCH(CH_2F)_2$ | $CH_3$ | $CH_3$ | $CH_3$ | H |
| F | CN | $SCH_2COOC_3H_7$ | $CH_3$ | $CH_3$ | $CH_3$ | H |
| F | CN | $SCHCOOC_2H_5$<br>\|<br>$CH_3$ | $CH_3$ | $CH_3$ | $CH_3$ | H |
| H | CN | $OCH_2CH{=}CHCH_3$ | $CH_3$ | $CH_3$ | $CH_3$ | H |
| H | CN | $OCH_2C{\equiv}CH$ | $CH_3$ | $CH_3$ | $CH_3$ | H |
| H | CN | $OCHC{\equiv}CH$<br>\|<br>$CH_3$ | $CH_3$ | $CH_3$ | $CH_3$ | H |
| H | CN | $OCH_2COOC_4H_9$ | $CH_3$ | $CH_3$ | $CH_3$ | H |
| H | CN | $OCHCOOC_2H_5$<br>\|<br>$CH_3$ | $CH_3$ | $CH_3$ | $CH_3$ | H |
| H | CN | $SCH_2CH{=}CHCl$ | $CH_3$ | $CH_3$ | $CH_3$ | H |
| H | CN | $SCH_2C{\equiv}CH$ | $CH_3$ | $CH_3$ | $CH_3$ | H |
| H | CN | $SC(CH_3)_2C{\equiv}CH$ | $CH_3$ | $CH_3$ | $CH_3$ | H |
| H | CN | $SCH_2COOC_2H_5$ | $CH_3$ | $CH_3$ | $CH_3$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| H | CN | $SCH(CH_3)COOCH_3$ | $CH_3$ | $CH_3$ | $CH_3$ | H |
| H | Cl | $SCH_2CH=CH_2$ | $CH_3$ | $CH_3$ | $CH_3$ | H |
| H | Cl | $SCH(CH_3)CH=CH_2$ | $CH_3$ | $CH_3$ | $CH_3$ | H |
| H | Cl | $SCH_2C≡CH$ | $CH_3$ | $CH_3$ | $CH_3$ | H |
| H | Cl | $SC(CH_3)_2C≡CH$ | $CH_3$ | $CH_3$ | $CH_3$ | H |
| H | Cl | $SCH_2C≡CCH_3$ | $CH_3$ | $CH_3$ | $CH_3$ | H |
| H | Cl | $SCH_2COOC_3H_7$ | $CH_3$ | $CH_3$ | $CH_3$ | H |
| H | Cl | $SCH(CH_3)COOC_2H_5$ | $CH_3$ | $CH_3$ | $CH_3$ | H |
| F | Cl | $SCH_2CH=CH_2$ | $CH_3$ | $CH_3$ | $CH_3$ | H |
| F | Cl | $SCH_2CH=CHCH_3$ | $CH_3$ | $CH_3$ | $CH_3$ | H |
| F | Cl | $SCH_2CH=CHCl$ | $CH_3$ | $CH_3$ | $CH_3$ | H |
| F | Cl | $SCH_2C≡CH$ | $CH_3$ | $CH_3$ | $CH_3$ | H |
| F | Cl | $SCH(CH_3)C≡CH$ | $CH_3$ | $CH_3$ | $CH_3$ | H |
| F | Cl | $SCH_2C≡CCH_3$ | $CH_3$ | $CH_3$ | $CH_3$ | H |
| F | Cl | $SCH_2CH_2CN$ | $CH_3$ | $CH_3$ | $CH_3$ | H |
| F | Cl | $SCH_2CH_2COOCH_3$ | $CH_3$ | $CH_3$ | $CH_3$ | H |
| F | Cl | $SCH(CH_2F)_2$ | $CH_3$ | $CH_3$ | $CH_3$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| F | Cl | $SCH_2COOC_4H_9$ | $CH_3$ | $CH_3$ | $CH_3$ | H |
| F | Cl | $SCH_2COO$ 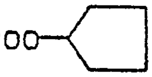 | $CH_3$ | $CH_3$ | $CH_3$ | H |
| F | Cl | $SCH_2COOCH_2$ 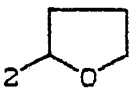 | $CH_3$ | $CH_3$ | $CH_3$ | H |
| F | Cl | $SCHCOOCH_3$<br>$CH_3$ | $CH_3$ | $CH_3$ | $CH_3$ | H |
| H | Cl | $SCH_2CH=CH_2$ | $CH_3$ | $CH_3$ | $C_2H_5$ | H |
| H | Cl | $SCH_2CH=CHCl$ | $CH_3$ | $CH_3$ | $C_2H_5$ | H |
| H | Cl | $SCH_2C\equiv CH$ | $CH_3$ | $CH_3$ | $C_2H_5$ | H |
| H | Cl | $SCHC\equiv CH$<br>$CH_3$ | $CH_3$ | $CH_3$ | $C_2H_5$ | H |
| H | Cl | $SCF_2CHFCl$ | $CH_3$ | $CH_3$ | $C_2H_5$ | H |
| H | Cl | $SCH(CH_2F)_2$ | $CH_3$ | $CH_3$ | $C_2H_5$ | H |
| H | Cl | $SCH_2CH_2CN$ | $CH_3$ | $CH_3$ | $C_2H_5$ | H |
| H | Cl | $SCH_2CH_2COOC_2H_5$ | $CH_3$ | $CH_3$ | $C_2H_5$ | H |
| H | Cl | $SCH_2COOC_4H_9$ | $CH_3$ | $CH_3$ | $C_2H_5$ | H |
| H | Cl | $SCH_2COO$ 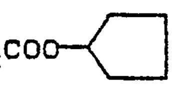 | $CH_3$ | $CH_3$ | $C_2H_5$ | H |
| H | Cl | $SCHCOOCH_3$<br>$CH_3$ | $CH_3$ | $CH_3$ | $C_2H_5$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| F | Cl | $SCH_2CH=CHCl$ | $CH_3$ | $CH_3$ | $C_2H_5$ | H |
| F | Cl | $SCH_2C\equiv CH$ | $CH_3$ | $CH_3$ | $C_2H_5$ | H |
| F | Cl | $SC(CH_3)_2C\equiv CH$ | $CH_3$ | $CH_3$ | $C_2H_5$ | H |
| F | Cl | $SCH_2CH_2COOCH_3$ | $CH_3$ | $CH_3$ | $C_2H_5$ | H |
| F | Cl | $SCH_2COO$ 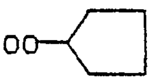 | $CH_3$ | $CH_3$ | $C_2H_5$ | H |
| F | Cl | $SCH(CH_2F)_2$ | $CH_3$ | $CH_3$ | $C_2H_5$ | H |
| F | Cl | $SCH_2CH_2COOCH_3$ | $CH_3$ | $CH_3$ | $C_2H_5$ | H |
| F | Cl | $SCHCOOCH_3$<br>$CH_3$ | $CH_3$ | $CH_3$ | $C_2H_5$ | H |
| H | CN | $OCH_2CH=CH_2$ | $CH_3$ | $CH_3$ | $C_2H_5$ | H |
| H | CN | $OCHF_2$ | $CH_3$ | $CH_3$ | $C_2H_5$ | H |
| H | CN | $OCF_2CHFCl$ | $CH_3$ | $CH_3$ | $C_2H_5$ | H |
| H | CN | $OCH_2C\equiv CH$ | $CH_3$ | $CH_3$ | $C_2H_5$ | H |
| H | CN | $SCH_2C\equiv CH$ | $CH_3$ | $CH_3$ | $C_2H_5$ | H |
| H | CN | $SCHC\equiv CH$<br>$CH_3$ | $CH_3$ | $CH_3$ | $C_2H_5$ | H |
| H | CN | $SCH_2COOC_3H_7$ | $CH_3$ | $CH_3$ | $C_2H_5$ | H |
| H | CN | $SCH_2COO$ 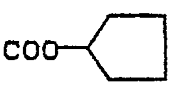 | $CH_3$ | $CH_3$ | $C_2H_5$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| H | CN | $SCHCOOC_2H_5$<br>$CH_3$ | $CH_3$ | $CH_3$ | $C_2H_5$ | H |
| F | CN | $OCH_2CH{=}CHCH_3$ | $CH_3$ | $CH_3$ | $C_2H_5$ | H |
| F | CN | $OCH_2C{\equiv}CH$ | $CH_3$ | $CH_3$ | $C_2H_5$ | H |
| F | CN | $OCH_2COOC_4H_9$ | $CH_3$ | $CH_3$ | $C_2H_5$ | H |
| F | CN | $O(CH_2CH_2O)_2C_2H_5$ | $CH_3$ | $CH_3$ | $C_2H_5$ | H |
| F | CN | $SCH_2CH{=}CH_2$ | $CH_3$ | $CH_3$ | $C_2H_5$ | H |
| F | CN | $SCH_2CH{=}CHCl$ | $CH_3$ | $CH_3$ | $C_2H_5$ | H |
| F | CN | $SCH_2CH_2CN$ | $CH_3$ | $CH_3$ | $C_2H_5$ | H |
| F | CN | $SCH_2COOC_2H_5$ | $CH_3$ | $CH_3$ | $C_2H_5$ | H |
| F | CN | $SCH_2CH_2COOCH_3$ | $CH_3$ | $CH_3$ | $C_2H_5$ | H |
| F | CN | $SCHCOOCH_3$<br>$CH_3$ | $CH_3$ | $CH_3$ | $C_2H_5$ | H |
| H | Cl | $SCH_2CH{=}CH_2$ | $CH_3$ | $CH_3$ | $C_3H_7$ | H |
| H | Cl | $SCH_2C{\equiv}CH$ | $CH_3$ | $CH_3$ | $C_3H_7$ | H |
| H | Cl | $SCHC{\equiv}CH$<br>$CH_3$ | $CH_3$ | $CH_3$ | $C_3H_7$ | H |
| H | Cl | $SCH_2COOC_4H_9$ | $CH_3$ | $CH_3$ | $C_3H_7$ | H |
| H | Cl | $SCH_2CH_2CN$ | $CH_3$ | $CH_3$ | $C_3H_7$ | H |
| H | Cl | $SCH_2CH_2COOCH_3$ | $CH_3$ | $CH_3$ | $C_3H_7$ | H |
| H | Cl | $SCH(CH_2F)_2$ | $CH_3$ | $CH_3$ | $C_3H_7$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| H | Cl | $SCHCOOCH_3$<br>$CH_3$ | $CH_3$ | $CH_3$ | $C_3H_7$ | H |
| F | Cl | $SCH_2CH{=}CH_2$ | $CH_3$ | $CH_3$ | $C_3H_7$ | H |
| F | Cl | $SCH_2CH{=}CHCl$ | $CH_3$ | $CH_3$ | $C_3H_7$ | H |
| F | Cl | $SCHC{\equiv}CH$<br>$CH_3$ | $CH_3$ | $CH_3$ | $C_3H_7$ | H |
| F | Cl | $SCH_2C{=}CH_2$<br>Cl | $CH_3$ | $CH_3$ | $C_3H_7$ | H |
| F | Cl | $SCH_2CH_2CN$ | $CH_3$ | $CH_3$ | $C_3H_7$ | H |
| F | Cl | $SCH(CH_2F)_2$ | $CH_3$ | $CH_3$ | $C_3H_7$ | H |
| F | Cl | $SCH_2COOC_3H_7$ | $CH_3$ | $CH_3$ | $C_3H_7$ | H |
| F | Cl | $SCH_2COO$ 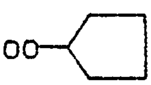 | $CH_3$ | $CH_3$ | $C_3H_7$ | H |
| F | Cl | $SCHCOOCH_3$<br>$CH_3$ | $CH_3$ | $CH_3$ | $C_3H_7$ | H |
| H | CN | $OCH_2CH{=}CH_2$ | $CH_3$ | $CH_3$ | $C_3H_7$ | H |
| H | CN | $OCH_2C{\equiv}CH$ | $CH_3$ | $CH_3$ | $C_3H_7$ | H |
| H | CN | $OCHF_2$ | $CH_3$ | $CH_3$ | $C_3H_7$ | H |
| H | CN | $OCF_2CHF_2$ | $CH_3$ | $CH_3$ | $C_3H_7$ | H |
| H | CN | $OCH(CH_2F)_2$ | $CH_3$ | $CH_3$ | $C_3H_7$ | H |
| H | CN | $OCH_2COOC_4H_9$ | $CH_3$ | $CH_3$ | $C_3H_7$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| H | CN | $OCH_2COOCH_2$ 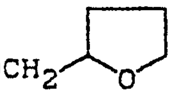 | $CH_3$ | $CH_3$ | $C_3H_7$ | H |
| H | CN | $SCH_2CH{=}CHCl$ | $CH_3$ | $CH_3$ | $C_3H_7$ | H |
| H | CN | $SCH_2C{\equiv}CH$ | $CH_3$ | $CH_3$ | $C_3H_7$ | H |
| H | CN | $SCH(CH_3)C{\equiv}CH$ | $CH_3$ | $CH_3$ | $C_3H_7$ | H |
| H | CN | $SCH_2COOC_2H_5$ | $CH_3$ | $CH_3$ | $C_3H_7$ | H |
| H | CN | $SCH(CH_3)COOCH_3$ | $CH_3$ | $CH_3$ | $C_3H_7$ | H |
| F | CN | $OCH_2CH{=}CH_2$ | $CH_3$ | $CH_3$ | $C_3H_7$ | H |
| F | CN | $OCH_2C{\equiv}CCH_3$ | $CH_3$ | $CH_3$ | $C_3H_7$ | H |
| F | CN | $OCH_2COOC_2H_5$ | $CH_3$ | $CH_3$ | $C_3H_7$ | H |
| F | CN | $SCH_2CH{=}CHCl$ | $CH_3$ | $CH_3$ | $C_3H_7$ | H |
| F | CN | $SCH_2C{\equiv}CCH_3$ | $CH_3$ | $CH_3$ | $C_3H_7$ | H |
| F | CN | $SCH_2COOCH_3$ | $CH_3$ | $CH_3$ | $C_3H_7$ | H |
| F | CN | $SCH_2CH_2COOCH_3$ | $CH_3$ | $CH_3$ | $C_3H_7$ | H |
| F | CN | $SCH_2COO$ 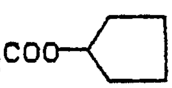 | $CH_3$ | $CH_3$ | $C_3H_7$ | H |
| F | CN | $SCH(CH_3)COOCH_3$ | $CH_3$ | $CH_3$ | $C_3H_7$ | H |
| H | Cl | $SCH_2CH{=}CH_2$ | $CH_3$ | $CH_3$ | $CH_3$ | $CH_3$ |

Tabelle 1 - Fortsetzung

| $R^1$ | $R^2$ | $R^3$ | $R^4$ | $R^5$ | $R^6$ | $R^7$ |
|---|---|---|---|---|---|---|
| H | Cl | $SCH_2CH=CHCl$ | $CH_3$ | $CH_3$ | $CH_3$ | $CH_3$ |
| H | Cl | $SCH_2C\equiv CH$ | $CH_3$ | $CH_3$ | $CH_3$ | $CH_3$ |
| H | Cl | $SCH_2C\equiv CCH_3$ | $CH_3$ | $CH_3$ | $CH_3$ | $CH_3$ |
| H | Cl | $SCHC\equiv CH$<br>\|<br>$CH_3$ | $CH_3$ | $CH_3$ | $CH_3$ | $CH_3$ |
| H | Cl | $SCH_2CH_2CN$ | $CH_3$ | $CH_3$ | $CH_3$ | $CH_3$ |
| H | Cl | $SCH_2COOC_4H_9$ | $CH_3$ | $CH_3$ | $CH_3$ | $CH_3$ |
| H | Cl | $SCHCOOC_2H_5$<br>\|<br>$CH_3$ | $CH_3$ | $CH_3$ | $CH_3$ | $CH_3$ |
| F | Cl | $SCH_2CH=CH_2$ | $CH_3$ | $CH_3$ | $CH_3$ | $CH_3$ |
| F | Cl | $SCH_2CH=CHCl$ | $CH_3$ | $CH_3$ | $CH_3$ | $CH_3$ |
| F | Cl | $SCH_2C\equiv CH$ | $CH_3$ | $CH_3$ | $CH_3$ | $CH_3$ |
| F | Cl | $SCH_2C\equiv CCH_3$ | $CH_3$ | $CH_3$ | $CH_3$ | $CH_3$ |
| F | Cl | $SCHC\equiv CH$<br>\|<br>$CH_3$ | $CH_3$ | $CH_3$ | $CH_3$ | $CH_3$ |
| F | Cl | $SCH(CH_2F)_2$ | $CH_3$ | $CH_3$ | $CH_3$ | $CH_3$ |
| F | Cl | $SCH_2CH_2CN$ | $CH_3$ | $CH_3$ | $CH_3$ | $CH_3$ |
| F | Cl | $SCH_2COOC_3H_7$ | $CH_3$ | $CH_3$ | $CH_3$ | $CH_3$ |
| F | Cl | $SCH_2COO$ 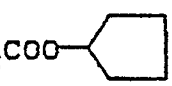 | $CH_3$ | $CH_3$ | $CH_3$ | $CH_3$ |

Tabelle 1 - Fortsetzung

| $R^1$ | $R^2$ | $R^3$ | $R^4$ | $R^5$ | $R^6$ | $R^7$ |
|---|---|---|---|---|---|---|
| F | Cl | $SCH(CH_3)COOCH_3$ | $CH_3$ | $CH_3$ | $CH_3$ | $CH_3$ |
| H | CN | $OCH_2CH=CHCH_3$ | $CH_3$ | $CH_3$ | $CH_3$ | $CH_3$ |
| H | CN | $OCH_2CH=CHCl$ | $CH_3$ | $CH_3$ | $CH_3$ | $CH_3$ |
| H | CN | $OCH_2C \equiv CH$ | $CH_3$ | $CH_3$ | $CH_3$ | $CH_3$ |
| H | CN | $OCHF_2$ | $CH_3$ | $CH_3$ | $CH_3$ | $CH_3$ |
| H | CN | $OCH_2COOC_4H_9$ | $CH_3$ | $CH_3$ | $CH_3$ | $CH_3$ |
| H | CN | $O(CH_2CH_2O)_2C_2H_5$ | $CH_3$ | $CH_3$ | $CH_3$ | $CH_3$ |
| H | CN | $SCH_2CH=CH_2$ | $CH_3$ | $CH_3$ | $CH_3$ | $CH_3$ |
| H | CN | $SCH_2C \equiv CH$ | $CH_3$ | $CH_3$ | $CH_3$ | $CH_3$ |
| H | CN | $SCH(CH_2F)_2$ | $CH_3$ | $CH_3$ | $CH_3$ | $CH_3$ |
| H | CN | $SCH_2CH_2CN$ | $CH_3$ | $CH_3$ | $CH_3$ | $CH_3$ |
| H | CN | $SCH_2COOC_2H_5$ | $CH_3$ | $CH_3$ | $CH_3$ | $CH_3$ |
| H | CN | $SCH(CH_3)COOCH_3$ | $CH_3$ | $CH_3$ | $CH_3$ | $CH_3$ |
| F | CN | $OCH_2CH=CH_2$ | $CH_3$ | $CH_3$ | $CH_3$ | $CH_3$ |
| F | CN | $OCH_2C \equiv CH$ | $CH_3$ | $CH_3$ | $CH_3$ | $CH_3$ |
| F | CN | $OCF_2CHFCl$ | $CH_3$ | $CH_3$ | $CH_3$ | $CH_3$ |
| F | CN | $OCH_2COOC_2H_5$ | $CH_3$ | $CH_3$ | $CH_3$ | $CH_3$ |
| F | CN | $OCH(CH_3)COOCH_3$ | $CH_3$ | $CH_3$ | $CH_3$ | $CH_3$ |

Tabelle 1 - Fortsetzung

| $R^1$ | $R^2$ | $R^3$ | $R^4$ | $R^5$ | $R^6$ | $R^7$ |
|---|---|---|---|---|---|---|
| F | CN | $O(CH_2CH_2O)_2CH_3$ | $CH_3$ | $CH_3$ | $CH_3$ | $CH_3$ |
| F | CN | $SCH_2CH=CH_2$ | $CH_3$ | $CH_3$ | $CH_3$ | $CH_3$ |
| F | CN | $SCH_2C\equiv CH$ | $CH_3$ | $CH_3$ | $CH_3$ | $CH_3$ |
| F | CN | $SCH_2COOC_3H_7$ | $CH_3$ | $CH_3$ | $CH_3$ | $CH_3$ |
| F | CN | $SCH(CH_3)COOC_2H_5$ | $CH_3$ | $CH_3$ | $CH_3$ | $CH_3$ |
| H | Cl | SH | $CH_3$ | $CH_3$ | $CH_3$ | H |
| H | Cl | SH | $CH_3$ | $CH_3$ | $C_2H_5$ | H |
| H | Cl | SH | $CH_3$ | $CH_3$ | $C_3H_7$ | H |
| H | Cl | SH | $CH_3$ | $CH_3$ | $-(CH_2)_5-$ | |
| H | Cl | SH | $CH_3$ | $CH_3$ | $CH_3$ | $CH_3$ |
| H | Cl | SH | $CH_3$ | $CH_3$ | H | H |
| F | Cl | SH | $CH_3$ | $CH_3$ | $CH_3$ | H |
| F | Cl | SH | $CH_3$ | $CH_3$ | $CH_3$ | $CH_3$ |
| F | Cl | SH | $CH_3$ | $CH_3$ | $C_2H_5$ | H |
| F | Cl | SH | $CH_3$ | $CH_3$ | $C_3H_7$ | H |
| F | Cl | SH | $CH_3$ | $CH_3$ | $-(CH_2)_5-$ | |
| F | Cl | SH | $CH_3$ | $CH_3$ | H | H |
| H | CN | OH | $CH_3$ | $CH_3$ | H | $CH_3$ |
| H | CN | SH | $CH_3$ | $CH_3$ | $CH_3$ | H |
| H | CN | OH | $CH_3$ | $CH_3$ | $C_2H_5$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| H | CN | OH | $CH_3$ | $CH_3$ | $C_3H_7$ | H |
| H | CN | SH | $CH_3$ | $CH_3$ | $C_2H_5$ | H |
| H | CN | SH | $CH_3$ | $CH_3$ | $C_3H_7$ | H |
| H | CN | OH | $CH_3$ | $CH_3$ | $-(CH_2)_5-$ | |
| H | CN | SH | $CH_3$ | $CH_3$ | $-(CH_2)_5-$ | |
| H | CN | OH | $CH_3$ | $CH_3$ | H | H |
| H | CN | SH | $CH_3$ | $CH_3$ | H | H |
| F | CN | OH | $CH_3$ | $CH_3$ | $CH_3$ | H |
| F | CN | SH | $CH_3$ | $CH_3$ | $CH_3$ | H |
| F | CN | OH | $CH_3$ | $CH_3$ | $C_2H_5$ | H |
| F | CN | SH | $CH_3$ | $CH_3$ | $C_2H_5$ | H |
| F | CN | OH | $CH_3$ | $CH_3$ | $C_3H_7$ | H |
| F | CN | SH | $CH_3$ | $CH_3$ | $C_3H_7$ | H |
| F | CN | OH | $CH_3$ | $CH_3$ | $-(CH_2)_5-$ | |
| F | CN | SH | $CH_3$ | $CH_3$ | $-(CH_2)_5-$ | |
| F | CN | OH | $CH_3$ | $CH_3$ | H | H |
| F | CN | SH | $CH_3$ | $CH_3$ | H | H |
| H | CN | OH | $-CH_2CHCH_2CH_2-$<br>\|<br>$CH_3$ | | $CH_3$ | H |
| H | CN | SH | $-CH_2CHCH_2CH_2-$<br>\|<br>$CH_3$ | | $CH_3$ | H |

<u>Tabelle 1</u> - Fortsetzung

| $R^1$ | $R^2$ | $R^3$ | $R^4$ | $R^5$ | $R^6$ | $R^7$ |
|---|---|---|---|---|---|---|
| H | CN | OH | $-CH_2\underset{\displaystyle CH_3}{\underset{\mid}{C}}HCH_2CH_2-$ | | $CH_3$ | $CH_3$ |
| H | CN | SH | $-CH_2\underset{\displaystyle CH_3}{\underset{\mid}{C}}HCH_2CH_2-$ | | $CH_3$ | $CH_3$ |
| H | CN | OH | $-CH_2\underset{\displaystyle CH_3}{\underset{\mid}{C}}HCH_2CH_2-$ | | H | H |
| H | CN | SH | $-CH_2\underset{\displaystyle CH_3}{\underset{\mid}{C}}HCH_2CH_2-$ | | H | H |
| H | CN | OH | $-CH_2\underset{\displaystyle CH_3}{\underset{\mid}{C}}HCH_2CH_2-$ | | $-(CH_2)_5-$ | |
| H | CN | SH | $-CH_2\underset{\displaystyle CH_3}{\underset{\mid}{C}}HCH_2CH_2-$ | | $-(CH_2)_5-$ | |
| H | CN | OH | $-(CH_2)_4-$ | | $CH_3$ | H |
| H | CN | SH | $-(CH_2)_4-$ | | $CH_3$ | H |
| H | CN | OH | $-(CH_2)_4-$ | | $CH_3$ | $CH_3$ |
| H | CN | SH | $-(CH_2)_4-$ | | $CH_3$ | $CH_3$ |
| H | CN | OH | $-(CH_2)_4-$ | | $-(CH_2)_5-$ | |
| H | CN | SH | $-(CH_2)_4-$ | | $-(CH_2)_5-$ | |
| H | CN | OH | $-(CH_2)_4-$ | | H | H |
| H | CN | SH | $-(CH_2)_4-$ | | H | H |
| F | CN | OH | $-(CH_2)_4-$ | | $CH_3$ | H |

Tabelle 1 - Fortsetzung

| $R^1$ | $R^2$ | $R^3$ | $R^4$ | $R^5$ | $R^6$ | $R^7$ |
|---|---|---|---|---|---|---|
| F | CN | SH | $-(CH_2)_4-$ | | $CH_3$ | H |
| F | CN | OH | $-(CH_2)_4-$ | | $CH_3$ | $CH_3$ |
| F | CN | SH | $-(CH_2)_4-$ | | $CH_3$ | $CH_3$ |
| F | CN | OH | $-(CH_2)_4-$ | | $-(CH_2)_5-$ | |
| F | CN | SH | $-(CH_2)_4-$ | | $-(CH_2)_5-$ | |
| F | CN | OH | $-(CH_2)_4-$ | | H | H |
| F | CN | SH | $-(CH_2)_4-$ | | H | H |
| F | CN | OH | $-CH_2\underset{\underset{CH_3}{\vert}}{C}HCH_2CH_2-$ | | $CH_3$ | H |
| F | CN | SH | $-CH_2\underset{\underset{CH_3}{\vert}}{C}HCH_2CH_2-$ | | $CH_3$ | H |
| F | CN | OH | $-CH_2\underset{\underset{CH_3}{\vert}}{C}HCH_2CH_2-$ | | $CH_3$ | $CH_3$ |
| F | CN | SH | $-CH_2\underset{\underset{CH_3}{\vert}}{C}HCH_2CH_2-$ | | $CH_3$ | $CH_3$ |
| F | CN | OH | $-CH_2\underset{\underset{CH_3}{\vert}}{C}HCH_2CH_2-$ | | $-(CH_2)_5-$ | |
| F | CN | SH | $-CH_2\underset{\underset{CH_3}{\vert}}{C}HCH_2CH_2-$ | | $-(CH_2)_5-$ | |
| F | CN | OH | $-CH_2\underset{\underset{CH_3}{\vert}}{C}HCH_2CH_2-$ | | H | H |

Tabelle 1 - Fortsetzung

| $R^1$ | $R^2$ | $R^3$ | $R^4$ | $R^5$ | $R^6$ | $R^7$ |
|---|---|---|---|---|---|---|
| F | CN | SH | $-CH_2CHCH_2CH_2-$<br>$CH_3$ | | H | H |
| H | Cl | SH | $-(CH_2)_4-$ | | $CH_3$ | H |
| H | Cl | SH | $-(CH_2)_4-$ | | $C_2H_5$ | H |
| H | Cl | SH | $-(CH_2)_4-$ | | $C_3H_7$ | H |
| H | Cl | SH | $-(CH_2)_4-$ | | $CH_3$ | $CH_3$ |
| H | Cl | SH | $-(CH_2)_4-$ | | $-(CH_2)_5-$ | |
| H | Cl | SH | $-(CH_2)_4-$ | | H | H |
| H | Cl | SH | $-CH_2CHCH_2CH_2-$<br>$CH_3$ | | $CH_3$ | H |
| H | Cl | SH | $-CH_2CHCH_2CH_2-$<br>$CH_3$ | | $C_2H_5$ | H |
| H | Cl | SH | $-CH_2CHCH_2CH_2-$<br>$CH_3$ | | $C_3H_7$ | H |
| H | Cl | SH | $-CH_2CHCH_2CH_2-$<br>$CH_3$ | | $CH_3$ | $CH_3$ |
| H | Cl | SH | $-CH_2CHCH_2CH_2-$<br>$CH_3$ | | $-(CH_2)_5-$ | |
| H | Cl | SH | $-CH_2CHCH_2CH_2-$<br>$CH_3$ | | H | H |
| F | Cl | SH | $-(CH_2)_4-$ | | $CH_3$ | H |

Tabelle 1 - Fortsetzung

| $R^1$ | $R^2$ | $R^3$ | $R^4$ $R^5$ | $R^6$ | $R^7$ |
|---|---|---|---|---|---|
| F | Cl | SH | $-(CH_2)_4-$ | $C_2H_5$ | H |
| F | Cl | SH | $-(CH_2)_4-$ | $C_3H_7$ | H |
| F | Cl | SH | $-(CH_2)_4-$ | $CH_3$ | $CH_3$ |
| F | Cl | SH | $-(CH_2)_4-$ | $-(CH_2)_5-$ | |
| F | Cl | SH | $-(CH_2)_4-$ | H | H |
| F | Cl | SH | $-CH_2CH(CH_3)CH_2CH_2-$ | $CH_3$ | H |
| F | Cl | SH | $-CH_2CH(CH_3)CH_2CH_2-$ | $C_2H_5$ | H |
| F | Cl | SH | $-CH_2CH(CH_3)CH_2CH_2-$ | $C_3H_7$ | H |
| F | Cl | SH | $-CH_2CH(CH_3)CH_2CH_2-$ | $CH_3$ | $CH_3$ |
| F | Cl | SH | $-CH_2CH(CH_3)CH_2CH_2-$ | $-(CH_2)_5-$ | |
| F | Cl | SH | $-CH_2CH(CH_3)CH_2CH_2-$ | H | H |

Verwendet man für das erfindungsgemäße Verfahren (a) beispielsweise 2-Acetyl-cyclohexen-1-carbonsäure und 2-Fluor-4-cyano-5-allyloxy-anilin als Ausgangsstoffe, so kann der Reaktionsablauf durch das folgende Formelschema wiedergegeben werden:

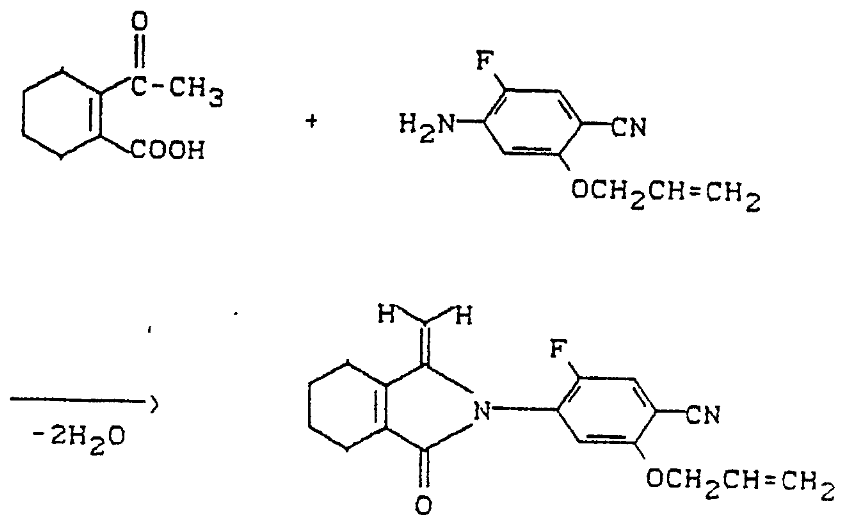


Verwendet man für das erfindungsgemäße Verfahren (b) beispielsweise N-(2-fluor-4-methyl-5-propargyloxy-phenyl)-3,4,5,6-tetrahydro-phthalimid und Propylmagnesiumbromid als Ausgangsstoffe, so kann der Reaktionsablauf durch das folgende Formelschema wiedergegeben werden:

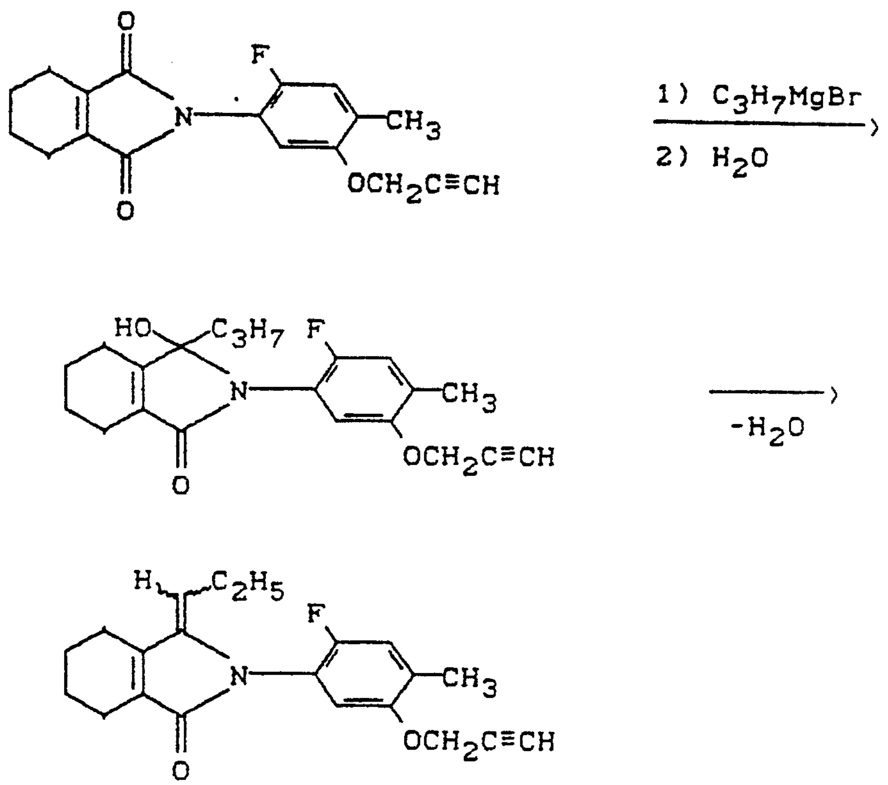


Verwendet man für das erfindungsgemäße Verfahren (c) beispielsweise 2-(2-fluor-4-cyano-5-hydroxyphenyl)-3-methylen-4,5,6,7-tetrahydro-2H-isoindol-1-on und Bromessigsäureethylester als Ausgangsstoffe, so kann der Reaktionsablauf durch das folgende Formelschema wiedergegeben werden:

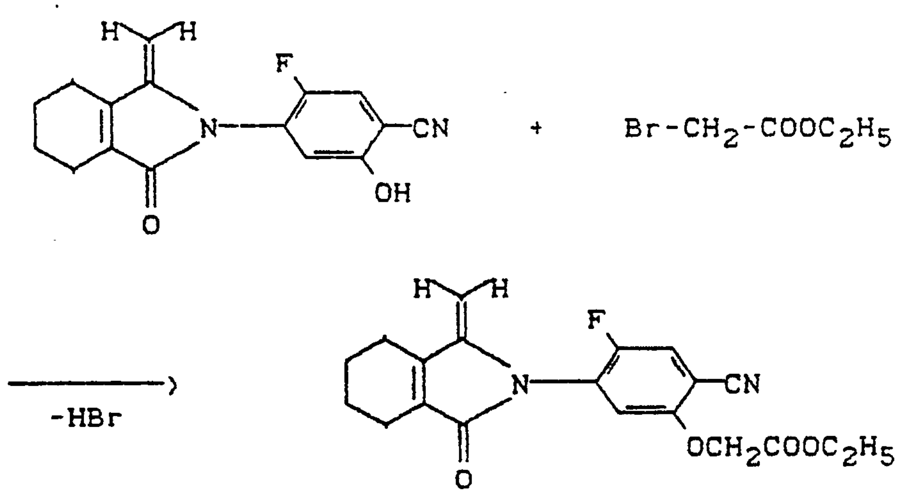


Verwendet man für das erfindungsgemäße Verfahren (d) beispielsweise 1-(2,5-Difluor-4-cyano-phenyl)-5-isopropyliden-3,4-dimethyl-1H-pyrrol-2-on und Allylalkohol als Ausgangsstoffe, so kann der Reaktionsablauf durch das folgende Formelschema wiedergegeben werden:

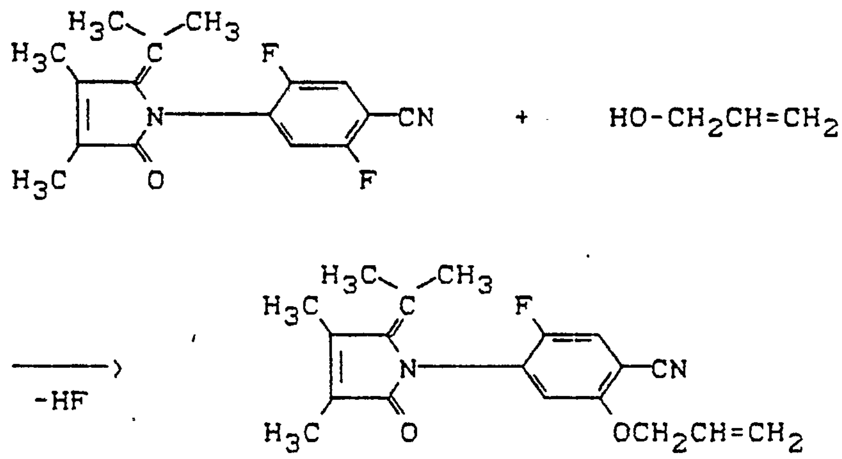


Die beim erfindungsgemäßen Verfahren (a) zur Herstellung von Verbindungen der Formel (I) als Ausgangsstoffe zu verwendenden Ketocarbonsäuren sind durch die Formel (II) allgemein definiert.

In Formel (II) haben $R^4$, $R^5$, $R^6$ und $R^7$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^4$, $R^5$, $R^6$ und $R^7$ angegeben wurden.

Die Ausgangsstoffe der Formel (II) sind bekannt und/oder können nach an sich bekannten Verfahren hergestellt werden (vgl. Helv.Chim.Acta 50 (1967), 798-807).

Die beim erfindungsgemäßen Verfahren (a) weiter als Ausgangsstoffe zu verwendenden Arylamine sind durch die Formel (III) allgemein definiert.

In Formel (III) haben $R^1$, $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$ und $R^3$ angegeben wurden.

Die Ausgangsstoffe der Formel (III) sind bekannt und/oder können nach an sich bekannten Verfahren hergestellt werden (vgl. EP-A 69855, EP-A 70389, EP-A75267, EP-A 290902).

Die beim erfindungsgemäßen Verfahren (b) zur Herstellung von Verbindungen der Formel (I) als Ausgangsstoffe zu verwendenden N-Aryl-imide sind durch die Formel (IV) allgemein definiert.

In Formel (IV) haben $R^1$, $R^2$, $R^3$, $R^4$ und $R^5$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$, $R^2$, $R^3$, $R^4$ und $R^5$ angegeben wurden.

Die Ausgangsstoffe der Formel (IV) sind bekannt und/oder können nach an sich bekannten Verfahren hergestellt werden (vgl. EP-A 61741, EP-A 83055, EP-A 126419, EP-A 211805, EP-A 303573).

Die beim erfindungsgemäßen Verfahren (b) weiter als Ausgangsstoffe zu verwendenden metallorganischen Verbindungen sind durch die Formel (V) allgemein definiert.

In Formel (V) haben $R^6$ und $R^7$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^6$ und $R^7$ angegeben wurden und

M steht vorzugsweise für Lithium oder die Gruppierung MgX, worin X für Chlor, Brom oder Jod steht.

Die Ausgangsstoffe der Formel (V) sind bekannte Synthesechemikalien

Die beim erfindungsgemäßen Verfahren (b) als Zwischenprodukte erhaltenen Hydroxyverbindungen der Formel (VI) sind neu und Gegenstand der vorliegenden Erfindung. Die Verbindungen der Formel (VI) sind in gewissem Umfang auch herbizid wirksam.

Die beim erfindungsgemäßen Verfahren (c) als Ausgangsstoffe zu verwendenden Verbindungen sind durch die Formel (I) mit der Maßgabe, daß $R^3$ für Hydroxy oder Mercapto steht, allgemein definiert.

In diesem Fall haben $R^1$, $R^2$, $R^4$, $R^5$, $R^6$ und $R^7$ vorzugsweise bzw. insbesondere diejenigen Bedeutungen, die oben im Rahmen der Beschreibung der erfindungsgemäßen Verbindungen der Formel (I) vorzugsweise bzw. als insbesondere bevorzugt für $R^1$, $R^2$, $R^4$, $R^5$, $R^6$ und $R^7$ angegeben wurden.

Die oben beschriebenen Ausgangsstoffe der Formel (I) für Verfahren (c) sind erfindungsgemäße, neue Verbindungen; sie können nach den erfindungsgemäßen Verfahren (a) und (b) hergestellt werden.

Die bei Verfahren (c) weiter als Ausgangsstoffe zu verwendenden Alkylierungsmittel sind durch die Formel (VII) allgemein definiert.

Vorzugsweise stehen in Formel (VII)

$R^8$ für gegebenenfalls substituiertes, geradkettiges oder verzweigtes Alkyl mit bis zu 8 Kohlenstoffatomen, wobei als Substituenten in Frage kommen: Fluor, Chlor, Brom, Cyano, Carboxy, $C_1$-$C_4$-Alkoxy, $C_1$-$C_4$-Alkoxy-$C_1$-$C_4$-alkoxy, $C_1$-$C_4$-Alkylthio, $C_1$-$C_4$-Alkyl-carbonyl, $C_1$-$C_4$-Alkoxy-carbonyl, $C_3$-$C_6$-Cycloalkyloxy-carbonyl, $C_3$-$C_6$-Cycloalkyl-$C_1$-$C_2$-alkoxy-carbonyl, $C_5$-$C_6$-Cycloalkenyloxy-carbonyl, $C_5$-$C_6$-Cycloalkenyl-$C_1$-$C_2$-alkoxy-carbonyl, Tetrahydrofurylmethoxycarbonyl; weiter steht $R^8$ vorzugsweise für jeweils gegebenenfalls substituiertes und gegebenenfalls verzweigtes Alkenyl, Alkinyl, Cycloalkyl mit jeweils bis zu 8 Kohlenstoffatomen, wobei als Substituenten in Frage kommen: Fluor, Chlor, Brom, Cyano, Carboxy, $C_1$-$C_4$-Alkoxy-carbonyl, ferner steht vorzugsweise

X für Chlor, Brom, Jod, Methylsulfonyloxy, Phenylsulfonyloxy, Tolylsulfonyloxy, Methoxysulfonyloxy oder Ethoxysulfonyloxy.

In Formel (VII) stehen insbesondere

$R^8$ für jeweils gegebenenfalls substituiertes Methyl, Ethyl, Propyl, Isopropyl, Butyl, Isobutyl, sec-Butyl, tert-Butyl, Pentyl, Isopentyl, sec-Pentyl, tert-Pentyl, Hexyl oder Isohexyl, wobei als Substituenten insbesondere in Frage kommen: Fluor, Chlor, Cyano, Carboxy, Methoxy, Ethoxy, Propoxy, Isopropoxy, Methoxyethoxy, Ethoxyethoxy, Methylthio, Ethylthio, Propylthio, Isopropylthio, Methylcarbonyl, Ethylcarbonyl, Propylcarbonyl, Isopropylcarbonyl, Butylcarbonyl, Isobutylcarbonyl, sec-Butylcarbonyl, tert-Butylcarbonyl, Methoxycarbonyl, Ethoxycarbonyl, Propoxycarbonyl, Isopropoxycarbonyl, Butoxycarbonyl, Isobutoxycarbonyl, sec-Butoxycarbonyl, tert-Butoxycarbonyl, Cyclopropyloxycarbonyl, Cyclopentyloxycarbonyl, Cyclohexyloxycarbonyl, Cyclopentenyloxycarbonyl, Cyclohexenyloxycarbonyl, Cyclopropylmethoxycarbonyl, Cyclopentylmethoxycarbonyl, Cyclohexylmethoxycarbonyl, Cyclopentenylmethoxycarbonyl, Cyclohexenylmethoxycarbonyl, Tetrahydrofurylmethoxycarbonyl;

weiter steht

$R^8$ insbesondere für Allyl, 1-Methyl-, 2-Methyl- und 3-Methyl-allyl, Propargyl, 1-Methyl-, 3-Methyl- und 1,1-Dimethyl-propargyl, Cyclopropyl, Cyclopentyl oder Cyclohexyl, welche jeweils gegebenenfalls durch Fluor und/oder Chlor substituiert sind, und

X für Chlor, Brom oder Jod.

Die Ausgangsstoffe der Formel (VII) sind bekannte organische Synthesechemikalien.

Die beim erfindungsgemäßen Verfahren (d) als Ausgangsstoffe zu verwendenden Verbindungen sind durch die Formel (I) mit der Maßgabe, daß $R^3$ für Halogen steht, allgemein definiert.

In diesem Fall haben $R^1$, $R^2$, $R^4$, $R^5$, $R^6$ und $R^7$ vorzugsweise bzw. insbesondere diejenigen Bedeutungen, die oben im Rahmen der Beschreibung der erfindungsgemäßen Verbindungen der Formel (I) vorzugsweise bzw. als insbesondere bevorzugt für $R^1$, $R^2$, $R^4$, $R^5$, $R^6$ und $R^7$ angegeben wurden und $R^3$ steht vorzugsweise für Fluor, Chlor oder Brom.

Die oben beschriebenen Ausgangsstoffe der Formel (I) für Verfahren (d) sind erfindungsgemäße, neue Verbindungen; sie können nach den erfindungsgemäßen Verfahren (a) oder (b) hergestellt werden.

Die bei Verfahren (d) weiter als Ausgangsstoffe zu verwendenden Verbindungen sind durch die Formel (VIII) allgemein definiert.

In Formel (VIII) hat $R^9$ vorzugsweise bzw. insbesondere diejenige Bedeutung, die oben im Zusammenhang mit der Beschreibung der erfindungsgemäßen Verbindungen der Formel (I) vorzugsweise bzw. als insbesondere bevorzugt für $R^3$ angegeben wurde, wobei die für Halogen angegebenen bevorzugten Bedeutungen ausgenommen sind. Bevorzugte Metallsalze hiervon sind die entsprechenden Lithium-, Natrium- und Kaliumsalze.

Die Ausgangsstoffe der Formel (VIII) sind bekannte organische Synthesechemikalien.

Das erfindungsgemäße Verfahren (a) zur Herstellung der neuen Verbindungen der Formel (I) wird gegebenenfalls unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Benzin, Benzol, Toluol, Xylol und Chlorbenzol. Verfahren (a) kann jedoch in vielen Fällen auch ohne Verwendung eines Verdünnungsmittels durchgeführt werden.

Das erfindungsgemäße Verfahren (a) wird vorzugsweise in Gegenwart eines sauren Katalysators durchgeführt. Als saure Katalysatoren kommen vor allem Protonensäuren wie Salzsäure, Schwefelsäure, Natrium- oder Kalium-hydrogensulfat, Methansulfonsäure, Benzolsulfonsäure und p-Toluolsulfonsäure in Betracht.

Die Reaktionstemperaturen können bei dem erfindungsgemäßen Verfahren (a) in einem größeren Bereich variiert werden. Im allgemeinen arbeitet man bei Temperaturen zwischen 20 °C und 200 °C, vorzugsweise bei Temperaturen zwischen 60 °C und 150 °C.

Das erfindungsgemäße Verfahren (a) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (a) setzt man auf 1 Mol Ketocarbonsäure der Formel (II) im allgemeinen zwischen 0,6 und 1,4 Mol, vorzugsweise zwischen 0,8 und 1,2 Mol Arylamin der Formel (III) ein.

Die Ausgangsstoffe der Formeln (II) und (III) werden im allgemeinen bei Raumtemperatur mit dem sauren Katalysator und gegebenenfalls mit dem Verdünnungsmittel vermischt und dann vorzugsweise bei erhöhter Temperatur bis zum Ende der Umsetzung gerührt. Die Aufarbeitung kann nach üblichen Methoden durchgeführt werden (vgl. die Herstellungsbeispiele).

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen praktisch alle inerten organischen Solventien in Betracht. Hierzu gehören vorzugsweise aliphatische und aromatische Kohlenwasserstoffe wie Pentan, Hexan, Heptan, Cyclohexan, Benzol und Toluol, sowie Ether wie Diethylether, Dipropylether, Diisopropylether, Dibutylether, Diisobutylether, Tetrahydrofuran, Dioxan und 1,2-Dimethoxyethan.

Die Reaktionstemperaturen können bei der ersten Stufe des erfindungsgemäßen Verfahrens (b) in einem größeren Bereich variiert werden. Im allgemeinen arbeitet man bei Temperaturen zwischen -50 °C und +100 °C, vorzugsweise bei Temperaturen zwischen 0 °C und +50 °C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) setzt man auf 1 Mol N-Aryl-imid der Formel (IV) im allgemeinen zwischen 1 und 3 Mol, vorzugsweise zwischen 1,1 und 2,5 Mol metallorganische Verbindung der Formel (V) ein.

Die Ausgangsstoffe der Formeln (IV) und (V) werden im allgemeinen bei Raumtemperatur oder unter Kühlen mit einem Lösungsmittel vermischt und bis zum Ende der Umsetzung gerührt, dann mit einer wäßrigen Säure, wie z.B. verdünnter Salzsäure, Schwefelsäure oder Essigsäure, versetzt und nach üblichen Methoden zur Isolierung der Verbindungen der Formel (VI) aufgearbeitet (vgl. Herstellungsbeispiele).

In einer zweiten Stufe wird dann die Dehydratisierung dieser Intermediate zu den entsprechenden Verbindungen der Formel (I) nach üblichen Methoden durchgeführt. Es werden hiebei vorzugsweise Verdünnungsmittel und saure Katalysatoren wie oben für Verfahren (a) angegeben eingesetzt. Die Reaktionskomponenten werden im allgemeinen analog Verfahren (a) umgesetzt und die Aufarbeitung wird nach üblichen Methoden durchgeführt (vgl. die Herstellungsbeispiele).

Das erfindungsgemäße Verfahren (c) zur Herstellung der neuen Verbindungen der Formel (I) wird vorzugsweise unter Verwendung, von Verdünnungsmitteln durchgeführt. Als Verdünnungsmittel kommen dabei neben Wasser praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methyl-isopropyl- und Methyl-isobutyl-keton, Ester wie Essigsäuremethylester und -ethylester, Nitrile

wie z. B. Acetonitril und Propionitril, Amide wie z. B. Dimethylformamid, Dimethylacetamid und N-Methylpyrrolidon sowie Dimethylsulfoxid, Tetramethylensulfon und Hexamethylphosphorsäuretriamid.

Als Säureakzeptoren können bei dem erfindungsgemäßen Verfahren (c) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate, -hydride und -alkoholate wie Natrium- und Kaliumcarbonat, Natrium-und Kaliumhydrid, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-un dec-7-en (DBU) und 1,4-Diazabicyclo-[2,2,2]-octan (DABCO).

Die Reaktionstemperaturen können bei dem erfindungsgemäßen Verfahren (c) in einem größeren Bereich variiert werden. Im allgemeinen arbeitet man bei Temperaturen zwischen -20 °C und +100 °C, vorzugsweise bei Temperaturen zwischen 0 °C und +80 °C.

Das erfindungsgemäße Verfahren (c) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (c) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (c) jeweils nach üblichen Methoden.

Das erfindungsgemäßen Verfahren (d) zur Herstellung der neuen Verbindungen der Formel (I) wird gegebenenfalls in Gegenwart eines Verdünnungsmittels durchgeführt. Es kommen hierbei vorzugsweise die gleichen Lösungsmittel in Betracht, die oben für das erfindungsgemäße Verfahren (c) angegeben sind.

Das erfindungsgemäße Verfahren (d) wird gegebenenfalls in Gegenwart eines Säureakzeptors durchgeführt. Es kommen hierbei vorzugsweise die gleichen Säurebindemittel in Betracht, die oben für das erfindungsgemäße Verfahren (c) angegeben sind.

Die Reaktionstemperaturen können bei dem erfindungsgemäßen Verfahren (d) in einem größeren Bereich variiert werden. Im allgemeinen arbeitet man bei Temperaturen zwischen 0 °C und 150 °C, vorzugsweise bei Temperaturen zwischen 10 °C und 100 °C.

Das erfindungsgemäße Verfahren (d) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (d) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei dem erfindungsgemäßen Verfahren (d) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Un kraut im weitesten, Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können

die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Verbindungen der Formel (I) eignen sich zur selektiven Bekämpfung von monokotylen und dikotylen Unkräutern in monokotylen und dikotylen Kulturen sowohl im Vorauflauf- als auch im Nachauflauf-Verfahren.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoff mengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungs mittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitsblaugen und Methylcellulose.

Es können in den Formulierungen Haftmittel wie Carboxy methylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl )-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkraut bekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 5-(2-Chlor-4-trifluormethyl-phenoxy)-2-nitrobenzoesäure (ACIFLUORFEN); Chloressigsäure-N-(methoxymethyl)-2,6-diethylanilid (ALACHLOR); Methyl-6,6-dimethyl-2,4-dioxo-3-[1-(2-propenyloxyamino)-butyliden]-cyclohexancarbonsäure (ALLOXYDIM); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); N-(Butoxymethyl)-2-chlor-N-(2,6-diethylphenyl)-acetamid (BUTACHLOR); Ethyl-2-{[(4-chlor-6-methoxy-2-pyrimidinyl)-aminocarbonyl]-aminosulfonyl}-benzoat (CHLORIMURON); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); exo-1-Methyl-4-(1-methylethyl)-2-(2-methylphenyl-methoxy)-7-oxabicyclo-(2,2,1)-heptan (CINMETHYLIN); 3,6-Dichlor-2-pyridincarbonsäure (CLOPYRALID); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl- oder deren Ethylester (DICLOFOP); 2-[(2-Chlorphenyl)-methyl]-4,4-dimethylisoxazolidin-3-on (DIMETHAZONE); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); 2-[4-(5-Trifluormethyl-2-pyridyloxy)-phenoxy]-propansäure oder deren Butylester (FLUAZIFOP); [(4-Amino-3,5-dichlor-6-fluor-2-pyridinyl)- oxy]-essigsäure bzw. deren 1-Methylheptylester (FLUROXYPYR); 5-(2-Chlor-4-trifluormethyl-phenoxy)-N-methylsulfonyl-2-nitrobenzamid (FOMESAFEN); 2-{4-[(3-Chlor-5-(trifluormethyl)-2-pyridinyl)-oxy]-phenoxy}-propansäure bzw. deren Ethylester (HALOXYFOP); 2-[4,5-Dihydro-4-methyl-4-isopropyl-5-oxo-(1H)-imidazol-2-yl]-5-ethylpyridin-3-carbonsäure (IMAZETHAPYR); 3,5-Diiod-4-hydroxybenzonitril (IOXYNIL); N,N-Dimethyl-N′-(4-isopropylphenyl)-harnstoff (ISOPROTURON); (2-Ethoxy-1-methyl-2-oxo-ethyl)-5-[2-chlor-4-(trifluormethyl)-phenoxy]-2-nitrobenzoat (LACTOFEN); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Chlor-N-(2,6-dimethylphenyl)-N-[(1H)-pyrazol-1-yl-methyl]-acetamid (METAZACHLOR); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); 4-(Di-n-propylamino)-3,5-dinitrobenzolsulfonamid (ORYZALIN); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); O-(6-Chlor-3-phenylpyridazin-4-yl)-S-octyl-thiocarbonat (PYRIDATE); 2-[4-(6-Chlor-chinoxalin-2-yl-oxy)-phenoxy]-propionsäureethylester (QUIZALOFOPETHYL); 2-[1-(Ethoxamino)-butyliden]-5-(2-ethylthiopropyl)-1,3-cyclohexadion (SETHOXYDIM); 2-Chlor-4,6-bis-(ethylamino)-1,3,5-triazin (SIMAZIN); 2,4-Bis-[N-ethylamino]-6-methylthio-1,3,5-triazin (SIMETRYNE); 4-Ethylamino-2-t-butylamino-6-methylthio-s-triazin (TERBUTRYNE); 3-[[[[(4-Methoxy-6-methyl-1,3,5-triazin-2-yl)-aminol-carbonyl]-amino]-sulfonyl]-thiophen-2-carbonsäure- methylester (THIAMETURON); S-[(4-Chlorphenyl)-methyl]-N,N-diethyl-thiocarbamat (THIOBENCARB); N,N-Diisopropyl-S-(2,3,3-trichlorallyl)-thiolcarbamat (TRIALLATE); 2,6-Dinitro-4-trifluormethyl-N,N-dipropylanilin (TRIFLURALIN). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,001 und 5 kg Wirkstoff pro Hektar Bodenfläche, vorzugsweise zwischen 0,005 und 3 kg pro ha.

Die Herstellung und die Verwendung der erfindungsgemäßen Wirkstoffe geht aus den nachfolgenden Beispielen hervor.

Herstellungsbeispiele:

Beispiel 1

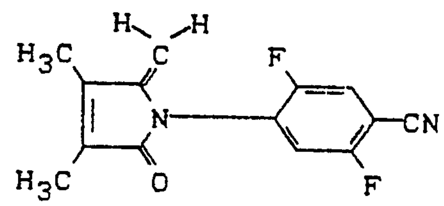

(Verfahren (a))

Eine Mischung aus 20,0 g (0,141 Mol) 2,3-Dimethyl-4-oxo-2-pentensäure, 21,7 g (0,141 Mol) 2,5-Difluor-4-cyanoanilin und 4,0 g (0,02 Mol) p-Toluolsulfonsäure wird 12 Stunden bei 110°C gerührt, wobei das freigesetzte Wasser am Wasserabscheider abgenommen wird. Dann wird abgekühlt, mit Methylenchlorid und 2N-Salzsäure geschüttelt, die organische Phase abgetrennt, mit gesättigter Natriumhydrogencarbonat-Lösung gewaschen und eingeengt. Der Rückstand wird chromatographisch (Kieselgel/Diethylether) gereinigt.

Man erhält 10,7 g (29% der Theorie) 1-(2,5-Difluor-4-cyano-phenyl)-3,4-dimethyl-5-methylen-pyrrol-(1H)2-on vom Schmelzpunkt 117°C.

Beispiel 2

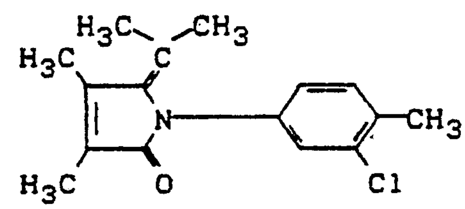

(Verfahren (b))

Vorprodukt: Beispiel VI-1

Eine Lösung von 22,1 g (0,15 Mol) Isopropylmagnesiumbromid in 150 ml Diethylether wird tropfenweise unter Rühren zu einer Lösung von 25 g (0,10 Mol) N-(3-Chlor-4-methyl-phenyl)-dimethylmaleinimid in 100 ml Tetrahydrofuran gegeben und die Mischung wird 6 Stunden bei 20°C gerührt. Dann wird mit 2N-Salzsäure angesäuert, die organische Phase abgetrennt, mit gesättigter Natriumhydrogencarbonat-Lösung gewaschen und mit Natriumsulfat getrocknet. Nach Filtration wird eingeengt, der Rückstand in Hexan aufgenommen und das ungelöste Ausgangsprodukt durch Filtration abgetrennt. Vom Filtrat wird das Lösungsmittel im Wasserstrahlvakuum sorgfältig abdestilliert.

Man erhält 20,9 g (71% der Theorie) 1-(3-Chlor-4-methylphenyl)-3,4-dimethyl-5-hydroxy-5-isopropyl-pyrrol-(1H)2-on als amorphen Rückstand.

$^1$H-NMR ($CDCl_3$, δ, ppm): 0,521 ($CH_3$),
0,544 ($CH_3$),
0,938 ($CH_3$),
0,962 ($CH_3$),
2,09 ($CH_3$).

Umsetzung zur Verbindung gemäß Beispiel 2:

14,3 g (48,7 mMol) 1-(3-Chlor-4-methylphenyl)-3,4-dimethyl-5-hydroxy-5-isopropyl-pyrrol-(1H)2-on werden zusammen mit 2,0 g (10 mMol) p-Toluolsulfonsäure und 100 ml Toluol zum Sieden erhitzt und das freigesetzte Wasser wird am Wasserabscheider entnommen. Nach 6 Stunden wird abgekühlt, mit gesättigter Natriumhydrogencarbonat-Lösung gewaschen, mit Natriumsulfat getrocknet und filtriert. Das Filtrat wird eingeengt und der Rückstand chromatographisch (Kieselgel/Diisopropylether) gereinigt.

Man erhält 8,0 g (60% der Theorie) 1-(3-Chlor-4-methylphenyl)-3,4-dimethyl-5-isopropyliden-pyrrol-(1H)2-on als amorphes Produkt.

$^1$H-NMR ($CDCl_3$, δ, ppm): 2,086 ($CH_3$).

Beispiel 3

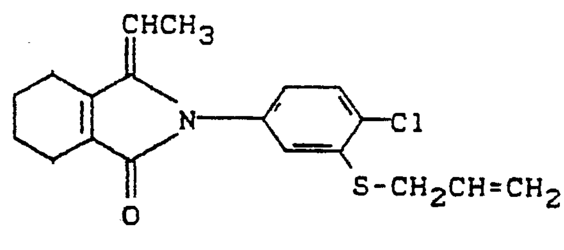

(Verfahren (c))

3,0 g (0,025 Mol) Allylbromid werden unter Rühren tropfenweise zu einer Mischung aus 7,5 g (0,025 Mol) 2-(4- Chlor-3-mercapto-phenyl)-3-ethyliden-4,5,6,7-tetrahydro-2H-isoindol-1-on, 1,4 g (0,025 Mol) Kaliumhydroxid, 10 ml Wasser und 50 ml Dimethylsulfoxid gegeben und das Gemisch wird noch 2 Stunden bei 50° C gerührt.

Nach Abkühlen wird mit 300 ml Wasser verdünnt und mit 300 ml Essigester geschüttelt. Die organische Phase wird abgetrennt, mit Wasser gewaschen, mit Natriumsulfat getrocknet und filtriert. Das Filtrat wird eingeengt und der Rückstand chromatographisch (Kieselgesäule; Eluens: Essigester/Cyclohexan 1:3) gereinigt.

Man erhält 1,8 g (21% der Theorie) 2-(3-Allylthio-4-chlor-phenyl)-3-ethyliden-4,5,6,7-tetrahydro-2H-isoindol-1-on vom Brechungsindex $n_D^{20}$ = 1,5945.

Beispiel 4

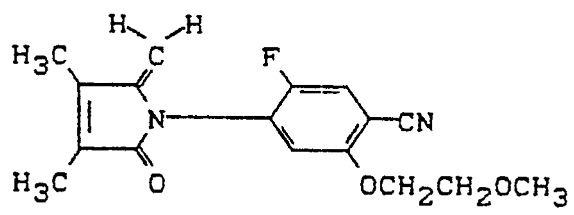

(Verfahren (d))

Zu 1 ml 2-Methoxy-ethanol werden nacheinander 0,17 g (6 mMol) Natriumhydrid (80%ig) und 0,60 g (2,3 mMol) 1-(2,5-Difluor-4-cyano-phenyl)-3,4-dimethyl-5-methylenpyrrol-(1H)2-on unter Rühren gegeben und das Gemisch wird 12 Stunden bei 20° C gerührt. Dann wird mit Wasser langsam auf etwa das 5fache Volumen verdünnt und das kristallin angefallene Produkt durch Absaugen isoliert.

Man erhält 0,50 g (68% der Theorie) 1-(2-Fluor-4-cyano-5-(2-methoxy-ethoxy)-phenyl)-3,4-dimethyl-5-methylenpyrrol-(1H)2-on vom Schmelzpunkt 78° C.

Analog zu den Beispielen 1 bis 4 und entsprechend der allgemeinen Beschreibung der erfindungsgemäßen Herstellungsverfahren können beispielsweise auch die in der nachstehenden Tabelle 2 aufgeführten Verbindungen der Formel (I) hergestellt werden.

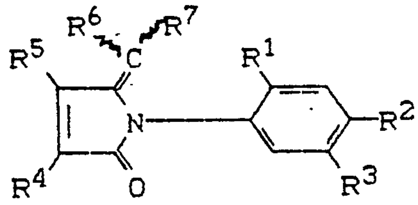

(I)

**Tabelle 2:** Herstellungsbeispiele für die Verbindungen der Formel (I)

| Bsp.-Nr. | $R^1$ | $R^2$ | $R^3$ | $R^4$ | $R^5$ | $R^6$ | $R^7$ | Physikal. Daten |
|---|---|---|---|---|---|---|---|---|
| 5 | F | Cl | $O(CH_2CH_2O)_2C_2H_5$ | $CH_3$ | $CH_3$ | H | H | $^1$H-NMR ($CDCl_3$, $\delta$, ppm): 1,96 ($CH_3$), 2,10 ($CH_3$) |
| 6 | F | Cl | $O(CH_2CH_2O)_2C_2H_5$ | $CH_3$ | $CH_3$ | $C_2H_5$ | H | $n_D^{20}$ : 1,5484 |
| 7 | F | Cl | $O(CH_2CH_2O)_2C_2H_5$ | $-(CH_2)_4-$ | | $CH_3$ | H | $n_D^{20}$ : 1,5530 |
| 8 | F | Cl | $O(CH_2CH_2O)_2C_2H_5$ | $CH_3$ | $CH_3$ | $CH_3$ | $CH_3$ | $n_D^{20}$ : 1,5485 |
| 9 | F | Cl | $O(CH_2CH_2O)_2C_2H_5$ | $-(CH_2)_4-$ | | $C_2H_5$ | H | $n_D^{20}$ : 1,5486 |
| 10 | F | Cl | $O(CH_2CH_2O)_2C_2H_5$ | $CH_3$ | $CH_3$ | $CH_3$ | H | $^1$H-NMR ($CDCl_3$, $\delta$, ppm): 1,42 ($CH_3$), 1,44 ($CH_3$) |
| 11 | F | Cl | $O(CH_2CH_2O)_2C_2H_5$ | $-(CH_2)_4-$ | | $CH_3$ | $CH_3$ | $n_D^{20}$ : 1,5619 |
| 12 | H | $CH_3$ | Cl | $CH_3$ | $CH_3$ | H | H | Fp.: 97° C |
| 13 | H | Cl | $SCH_2C{\equiv}CH$ | $-(CH_2)_4-$ | | H | H | $n_D^{20}$ : 1,6247 |

Tabelle 2 - Fortsetzung

| Bsp.-Nr. | $R^1$ | $R^2$ | $R^3$ | $R^4$ | $R^5$ | $R^6$ | $R^7$ | Physikal. Daten |
|---|---|---|---|---|---|---|---|---|
| 14 | H | Cl | $SCH_2CH=CH_2$ | $-(CH_2)_4-$ | | H | H | $n_D^{20}$: 1,6228 |
| 15 | H | Cl | $SCH_2C\equiv CH$ | $-(CH_2)_4-$ | | $CH_3$ | H | $n_D^{20}$: 1,6160 |
| 16 | F | CN | F | $-(CH_2)_4-$ | | H | H | Fp.: 124° C |
| 17 | F | CN | $OCH_2C\equiv CH$ | $-(CH_2)_4-$ | | H | H | Fp.: 146° C |
| 18 | H | Cl | SH | $-(CH_2)_4-$ | | $CH_3$ | H | Fp.: 85° C |
| 19 | H | Cl | SH | $-(CH_2)_4-$ | | H | H | Fp.: 70° C |
| 20 | F | CN | $OCH_2CH=CH_2$ | $-(CH_2)_4-$ | | H | H | Fp: 117° C |
| 21 | H | Cl | Cl | $CH_3$ | $CH_3$ | H | H | Fp.: 120° C |
| 22 | F | Cl | $O(CH_2CH_2O)_2C_2H_5$ | $-(CH_2)_4-$ | | H | H | Fp.: 77° C |
| 23 | H | CN | $OCH_2C\equiv CH$ | $-(CH_2)_4-$ | | H | H | Fp.: 165° C |
| 24 | H | CN | $OCH_2CH=CH_2$ | $-(CH_2)_4-$ | | H | H | Fp.: 105° C |
| 25 | F | CN | $OCH_2CH=CH_2$ | $-(CH_2)_4-$ | | H | H | Fp.: 117° C |
| 26 | F | CN | $OCH_2CH_2OCH_3$ | $-(CH_2)_4-$ | | H | H | Fp.: 88° C |

Anwendungsbeispiele:

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzübereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 1000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:
0 % = keine Wirkung (wie unbehandelte Kontrolle)
100 % = totale Vernichtung

In diesem Test zeigen beispielsweise die Verbindungen gemäß den Herstellungsbeispielen 5, 6, 7 und 8 bei guter Verträglichkeit gegenüber Nutzpflanzen, wie z.B. Weizen und Raps, sehr starke Wirkung gegen Unkräuter, wie z.B. Amaranthus, Galium, Protulaca und Veronica.

Beispiel B

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigen beispielsweise die Verbindungen gemäß den Herstellungsbeispielen 5, 6, 7, 9, 11 und 22 bei guter Verträglichkeit gegenüber Kulturpflanzen, wie z.B. Weizen, Mais und Soja, starke Wirkung gegenüber Unkräutern, wie z.B. Cynadon, Setaria, Abutilon, Amaranthus, Chenopadium und Galium.

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche

werden der Blattfall und das Austrocknen der Blätter im Vergleich zu den Kontrollpflanzen bonitiert.

In diesem Test zeigen beispielsweise die Wirkstoffe gemäß den Herstellungsbeispielen (7), (9), (11) und (22) ein sehr starkes Austrocknen der Blätter und sehr starken Blattfall.

## Ansprüche

1. N-Aryl-Stickstoffheterocyclen der Formel (I),

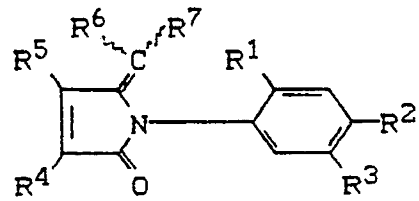

(I)

in welcher
$R^1$ für Wasserstoff oder Halogen steht,
$R^2$ für Cyano, Nitro, Fluor, Chlor, Brom, Jod, Alkyl, Halogenalkyl, Alkoxy, Halogenalkoxy, Alkylthio oder Halogenalkylthio steht,
$R^3$ für Halogen, Hydroxy, Mercapto oder für einen jeweils gegebenenfalls substituierten Rest aus der Reihe Alkoxy, Alkenyloxy, Alkinyloxy, Cycloalkyloxy, Alkylthio, Alkenylthio, Alkinylthio oder Cycloalkylthio steht,
$R^4$ für Wasserstoff, Halogen oder Alkyl steht,
$R^5$ für Wasserstoff, Halogen, Alkyl oder zusammen mit $R^4$ für Alkandiyl steht,
$R^6$ für Wasserstoff oder Alkyl steht und
$R^7$ für Wasserstoff oder Alkyl oder zusammen mit $R^6$ für Alkandiyl steht,
mit der Maßgabe, daß $R^2$ nur dann für Chlor steht, wenn entweder
(a) $R^1$ für Wasserstoff steht oder wenigstens einer der Reste $R^4$, $R^5$, $R^6$, $R^7$ für Alkyl steht und gleichzeitig
$R^3$ für Halogen, Mercapto oder für einen jeweils gegebenenfalls substituierten Rest aus der Reihe Alkylthio, Alkenylthio, Alkinylthio, Cycloalkylthio steht, oder
(b) $R^3$ für Halogen steht oder (in der Bedeutung "gegebenenfalls substituiertes Alkoxy") für Alkoxyalkoxyalkoxy steht.

2. N-Aryl-Stickstoffheterocyclen der Formel (I) gemäß Anspruch 1, in welcher
$R^1$ für Wasserstoff oder Fluor steht,
$R^2$ für Cyano, Nitro, Fluor, Chlor, Brom, Methyl, Ethyl, Methoxy, Ethoxy, Methylthio, Ethylthio, Halogenalkyl mit 1 oder 2 Kohlenstoffatomen und 1 bis 5 Halogenatomen Halogenalkoxy mit 1 oder 2 Kohlenstoffatomen und 1 bis 5 Halogenatomen oder Halogenalkylthio mit 1 oder 2 Kohlenstoffatomen und 1 bis 5 Halogenatomen steht,
$R^3$ für Fluor, Chlor, Brom, Hydroxy, Mercapto, für jeweils gegebenenfalls substituiertes, geradkettiges oder verzweigtes Alkoxy oder Alkylthio mit jeweils bis zu 8 Kohlenstoffatomen steht, wobei als Substituenten im Alkylteil jeweils in Frage kommen: Fluor, Chlor, Brom, Cyano, Carboxy, $C_1$-$C_4$-Alkoxy, $C_1$-$C_4$-Alkoxy-$C_1$-$C_4$-alkoxy, $C_1$-$C_4$-Alkylthio, $C_1$-$C_4$-Alkyl-carbonyl, $C_1$-$C_4$-Alkoxy-carbonyl, $C_3$-$C_6$-Cycloalkyloxy-carbonyl, $C_3$-$C_6$-Cycloalkyl-$C_1$-$C_2$-alkoxy-carbonyl, $C_5$-$C_6$-Cycloalkenyloxy-carbonyl, $C_5$-$C_6$-Cycloalkenyl-$C_1$-$C_2$-alkoxy-carbonyl, Tetrahydrofurylmethoxy-carbonyl; weiterhin
$R^3$ für jeweils gegebenenfalls substituiertes und jeweils gegebenenfalls verzweigtes Alkenyloxy, Alkinyloxy, Cycloalkyloxy, Alkenylthio, Alkinylthio oder Cycloalkylthio mit jeweils bis zu 8 Kohlenstoffatomen steht, wobei als Substituenten jeweils in Frage kommen: Fluor, Chlor, Brom, Cyano, Carboxy, $C_1$-$C_4$-Alkoxycarbonyl;
$R^4$ für Wasserstoff, Chlor oder für geradkettiges oder verzweigtes Alkyl mit bis 4 Kohlenstoffatomen steht,
$R^5$ für Wasserstoff, Chlor, geradkettiges oder verzweigtes Alkyl mit 1 bis 4 Kohlenstoffatomen steht oder $R^5$ zusammen mit $R^4$ für geradkettiges oder verzweigtes Alkandiyl mit 3 bis 6 Kohlenstoffatomen steht,
$R^6$ für Wasserstoff oder geradkettiges oder verzweigtes Alkyl mit 1 bis 4 Kohlenstoffatomen steht und
$R^7$ für Wasserstoff oder geradkettiges oder verzweigtes Alkyl mit 1 bis 4 Kohlenstoffatomen steht oder $R^7$ zusammen mit $R^6$ für geradkettiges oder verzweigtes Alkandiyl mit 2 bis 7 Kohlenstoffatomen steht,
mit der Maßgabe, daß $R^2$ nur dann für Chlor steht, wenn entweder
(a) $R^1$ für Wasserstoff steht oder wenigstens einer der Reste $R^4$, $R^5$, $R^6$, $R^7$ für Alkyl steht und

gleichzeitig
$R^3$ für Halogen, Mercapto oder für einen jeweils gegebenenfalls substituierten Rest aus der Reihe Alkylthio, Alkenylthio, Alkinylthio, Cycloalkylthio steht, oder

(b) $R^3$ für Halogen steht oder (in der Bedeutung "gegebenenfalls substituiertes Alkoxy") für Alkoxyalkoxyalkoxy steht.

3. Verfahren zur Herstellung von N-Aryl-Stickstoffheterocyclen der Formel (I),

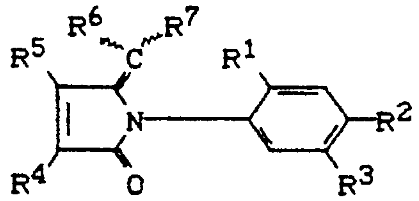

(I)

in welcher
$R^1$ für Wasserstoff oder Halogen steht,
$R^2$ für Cyano, Nitro, Fluor, Chlor, Brom, Jod, Alkyl, Halogenalkyl, Alkoxy, Halogenalkoxy, Alkylthio oder Halogenalkylthio steht,
$R^3$ für Halogen, Hydroxy, Mercapto oder für einen jeweils gegebenenfalls substituierten Rest aus der Reihe Alkoxy, Alkenyloxy, Alkinyloxy, Cycloalkyloxy, Alkylthio, Alkenylthio, Alkinylthio oder Cycloalkylthio steht,
$R^4$ für Wasserstoff, Halogen oder Alkyl steht,
$R^5$ für Wasserstoff, Halogen, Alkyl oder zusammen mit $R^4$ für Alkandiyl steht,
$R^6$ für Wasserstoff oder Alkyl steht und
$R^7$ für Wasserstoff oder Alkyl oder zusammen mit $R^6$ für Alkandiyl steht,
mit der Maßgabe, daß $R^2$ nur dann für Chlor steht, wenn entweder
(a) $R^1$ für Wasserstoff steht oder wenigstens einer der Reste $R^4$, $R^5$, $R^6$, $R^7$ für Alkyl steht und gleichzeitig
$R^3$ für Halogen, Mercapto oder für einen jeweils gegebenenfalls substituierten Rest aus der Reihe Alkylthio, Alkenylthio, Alkinylthio, Cycloalkylthio steht, oder
(b) $R^3$ für Halogen steht oder (in der Bedeutung "gegebenenfalls substituiertes Alkoxy") für Alkoxyalkoxyalkoxy steht,
dadurch gekennzeichnet, daß man

(a) Ketocarbonsäuren der allgemeinen Formel (II)

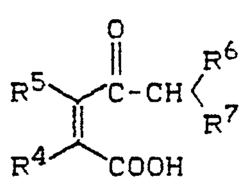

(II)

in welcher
$R^4$, $R^5$, $R^6$ und $R^7$ die oben angegebenen Bedeutungen haben,
mit Arylaminen der allgemeinen Formel (III)

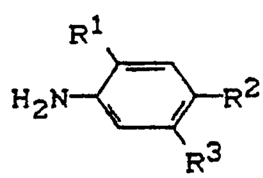

(III)

in welcher
$R^1$, $R^2$ und $R^3$ die oben angegebenen Bedeutungen haben,
gegebenenfalls in Gegenwart eines sauren Katalysators und gegebenenfalls in Gegenwart eines Verdünnungsmittels umsetzt, oder daß man

(b) N-Aryl-imide der allgemeinen Formel (IV)

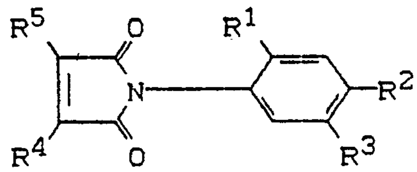

(IV)

in welcher
$R^1$, $R^2$, $R^3$, $R^4$ und $R^5$ die oben angegebenen Bedeutungen haben,
in einer ersten Stufe mit metallorganischen Verbindungen der allgemeinen Formel (V)

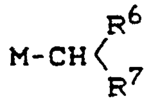

(V)

in welcher
$R^6$ und $R^7$ die oben angegebenen Bedeutungen haben
und
M für Lithium oder die Gruppierung MgX steht, worin
X für Chlor, Brom oder Jod steht,
gegebenenfalls in Gegenwart eines Verdünnungsmittels umsetzt und die nach Behandeln mit einer wäßrigen Säure erhaltenen Hydroxyverbindungen der allgemeinen Formel (VI)

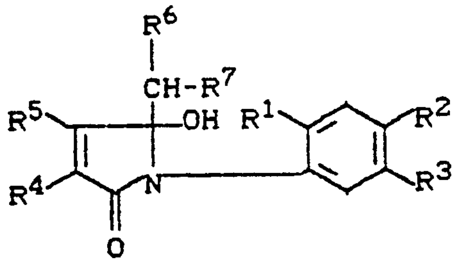

(VI)

in welcher
$R^1$, $R^2$, $R^3$, $R^4$, $R^5$, $R^6$ und $R^7$ die oben angegebenen Bedeutungen haben,
in einer zweiten Stufe dehydratisiert, oder daß man

(c) für den Fall, daß in der Formel (I)

$R^3$ für einen gegebenenfalls substituierten Rest aus der Reihe Alkoxy, Alkenyloxy, Alkinyloxy, Cycloalkyloxy, Alkylthio, Alkenylthio, Alkinylthio, Cycloalkylthio steht und
$R^1$, $R^2$, $R^4$, $R^5$, $R^6$ und $R^7$ die oben angegebenen Bedeutungen haben,
Verbindungen der Formel (I),
in welcher
$R^3$ für Hydroxy oder Mercapto steht und
$R^1$, $R^2$, $R^4$, $R^5$, $R^6$ und $R^7$ die oben angegebenen Bedeutungen haben,
mit Alkylierungsmitteln der Formel (VII)
$R^8$-X (VII)
in welcher
$R^8$ für jeweils gegebenenfalls substituiertes Alkyl, Alkenyl, Alkinyl oder Cycloalkyl steht und
X für eine nucleophile Abgangssgruppe steht,
gegebenenfalls in Gegenwart eines Säureakzeptors und gegebenenfalls in Gegenwart eines Verdünnungsmittels umsetzt, oder daß man

(d) für den Fall, daß in der Formel (I)

$R^3$ für Hydroxy, Mercapto oder für einen jeweils gegebenenfalls substituierten Rest aus der Reihe Alkoxy, Alkenyloxy, Alkinyloxy, Cycloalkyloxy, Alkylthio, Alkenylthio, Alkinylthio, Cycloalkylthio steht und
$R^1$, $R^2$, $R^4$, $R^5$, $R^6$ und $R^7$ die oben angegebenen Bedeutungen haben,
Verbindungen der Formel (I), in welcher

$R^3$ für Halogen steht und
$R^1$, $R^2$, $R^4$, $R^5$, $R^6$ und $R^7$ die oben angegebenen Bedeutungen haben,
mit Verbindungen der Formel (VIII)
H-$R^9$ (VIII)
in welcher
$R^9$ für Hydroxy, Mercapto oder für einen gegebenenfalls substituierten Rest aus der Reihe Alkoxy, Alkenyloxy, Alkinyloxy, Cycloalkyloxy, Alkylthio, Alkenylthio, Alkinylthio und Cycloalkylthio steht
oder mit Metallsalzen von Verbindungen der Formel (VIII)
gegebenenfalls in Gegenwart eines Säureakzeptors und gegebenenfalls in Gegenwart eines Verdünnungsmittels umsetzt.

4. Herbizide und pflanzenwuchsregulierende Mittel, gekenzeichnet durch einen Gehalt an mindestens einem N-Aryl-Stickstoffheterocyclus der Formel (I) gemäß Anspruch 1 oder 3.

5. Verfahren zur Bekämpfung von unerwünschten Pflanzen oder zur Regulierung des Pflanzenwachstums, dadurch gekennzeichnet, daß man N-Aryl-Stickstoffheterocyclen der Formel (I) gemäß Anspruch 1 oder 3 auf die unerwünschten Pflanzen und/oder ihren Lebensraum einwirken läßt.

6. Verwendung von N-Aryl-Stickstoffheterocyclen der Formel (I) gemäß Anspruch 1 oder 3 zur Bekämpfung von unerwünschten Pflanzen und/oder als Pflanzenwuchsregulatoren.

7. Verfahren zur Herstellung von herbiziden und/oder pflanzenwuchsregulierenden Mitteln, dadurch gekennzeichnet, daß man N-Aryl-Stickstoffheterocyclen der Formel (I) gemäß Anspruch 1 oder 3 mit Streckmitteln und/oder oberflächenaktiven Substanzen vermischt.

8. N-Aryl-Stickstoffheterocyclen der Formel (VI),

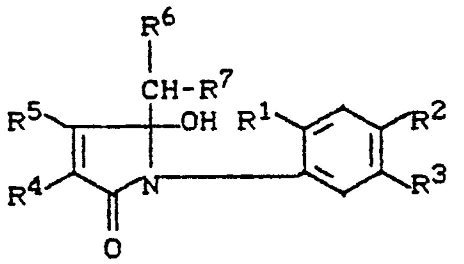

(VI)

in welcher
$R^1$ für Wasserstoff oder Halogen steht,
$R^2$ für Cyano, Nitro, Fluor, Chlor, Brom, Jod, Alkyl, Halogenalkyl, Alkoxy, Halogenalkoxy, Alkylthio oder Halogenalkylthio steht,
$R^3$ für Halogen, Hydroxy, Mercapto oder für einen jeweils gegebenenfalls substituierten Rest aus der Reihe Alkoxy, Alkenyloxy, Alkinyloxy, Cycloalkyloxy, Alkylthio, Alkenylthio, Alkinylthio oder Cycloalkylthio steht,
$R^4$ für Wasserstoff, Halogen oder Alkyl steht,
$R^5$ für Wasserstoff, Halogen, Alkyl oder zusammen mit $R^4$ für Alkandiyl steht,
$R^6$ für Wasserstoff oder Alkyl steht und
$R^7$ für Wasserstoff oder Alkyl oder zusammen mit $R^6$ für Alkandiyl steht,
mit der Maßgabe, daß $R^2$ nur dann für Chlor steht, wenn entweder

(a) $R^1$ für Wasserstoff steht oder wenigstens einer der Reste $R^4$, $R^5$, $R^6$, $R^7$ für Alkyl steht und gleichzeitig
$R^3$ für Halogen, Mercapto oder für einen jeweils gegebenenfalls substituierten Rest aus der Reihe Alkylthio, Alkenylthio, Alkinylthio, Cycloalkylthio steht, oder

(b) $R^3$ für Halogen steht oder (in der Bedeutung "gegebenenfalls substituiertes Alkoxy") für Alkoxyalkoxyalkoxy steht.

9. Verfahren zur Herstellung von Verbindungen der Formel (VI),

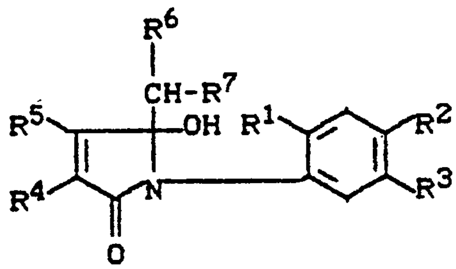

(VI)

in welcher
$R^1$ für Wasserstoff oder Halogen steht,
$R^2$ für Cyano, Nitro, Fluor, Chlor, Brom, Jod, Alkyl, Halogenalkyl, Alkoxy, Halogenalkoxy, Alkylthio oder Halogenalkylthio steht,
$R^3$ für Halogen, Hydroxy, Mercapto oder für einen jeweils gegebenenfalls substituierten Rest aus der Reihe Alkoxy, Alkenyloxy, Alkinyloxy, Cycloalkyloxy, Alkylthio, Alkenylthio, Alkinylthio oder Cycloalkylthio steht,
$R^4$ für Wasserstoff, Halogen oder Alkyl steht,
$R^5$ für Wasserstoff, Halogen, Alkyl oder zusammen mit $R^4$ für Alkandiyl steht,
$R^6$ für Wasserstoff oder Alkyl steht und
$R^7$ für Wasserstoff oder Alkyl oder zusammen mit $R^6$ für Alkandiyl steht,
mit der Maßgabe, daß $R^2$ nur dann für Chlor steht, wenn entweder
(a) $R^1$ für Wasserstoff steht oder wenigstens einer der Reste $R^4$, $R^5$, $R^6$, $R^7$ für Alkyl steht und gleichzeitig $R^3$ für Halogen, Mercapto oder für einen jeweils gegebenenfalls substituierten Rest aus der Reihe Alkylthio, Alkenylthio, Alkinylthio, Cycloalkylthio steht, oder
(b) $R^3$ für Halogen steht oder (in der Bedeutung "gegebenenfalls substituiertes Alkoxy") für Alkoxyalkoxyalkoxy steht,
dadurch gekennzeichnet, daß man N-Aryl-imide der allgemeinen Formel (IV)

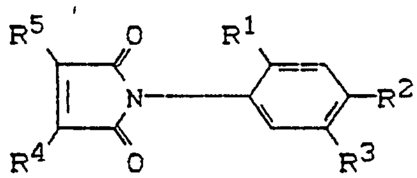

(IV)

in welcher
$R^1$, $R^2$, $R^3$, $R^4$ und $R^5$ die oben angegebenen Bedeutungen haben,
mit metallorganischen Verbindungen der allgemeinen Formel (V)

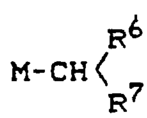

(V)

in welcher
$R^6$ und $R^7$ die oben angegebenen Bedeutungen haben und
M für Lithium oder die Gruppierung MgX steht, worin
X für Chlor, Brom oder Jod steht,
gegebenenfalls in Gegenwart eines Verdünnungsmittels umsetzt.

Europäisches Patentamt

# EUROPÄISCHER RECHERCHENBERICHT

Nummer der Anmeldung

EP 90110853.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 5) |
|---|---|---|---|
| D,A | EP - A2 - 0 305 333 (CIBA-GEIGY AG) * Zusammenfassung; Patentansprüche 3,4,8-10,12,13 * -- | 1,3-6 | C 07 D 207/44<br>C 07 D 209/46<br>A 01 N 43/36<br>A 01 N 43/38 |
| D,A | US - A - 3 992 189 (STEVEN JEROME GODDARD) * Zusammenfassung * ---- | 1,4,5 | |

RECHERCHIERTE SACHGEBIETE (Int Cl 5)

C 07 D 207/00
C 07 D 209/00
A 01 N

Der vorliegende Recherchenbericht wurde für alle Patentansprüche erstellt

| Recherchenort | Abschlußdatum der Recherche | Prüfer |
|---|---|---|
| WIEN | 08-08-1990 | HEIN |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument